(19) Europäisches Patentamt
European Patent Office
Office européen des brevets

(11) EP 2 133 094 A1

(12) **EUROPEAN PATENT APPLICATION**
published in accordance with Art. 153(4) EPC

(43) Date of publication:
**16.12.2009 Bulletin 2009/51**

(21) Application number: **08721328.6**

(22) Date of filing: **05.03.2008**

(51) Int Cl.:
***A61K 45/06*** *(2006.01)* ***A61K 31/198*** *(2006.01)*
***A61K 31/337*** *(2006.01)* ***A61K 31/5375*** *(2006.01)*
***A61K 31/5377*** *(2006.01)* ***A61K 38/00*** *(2006.01)*
***A61K 39/395*** *(2006.01)* ***A61P 35/00*** *(2006.01)*
***A61P 35/02*** *(2006.01)* ***A61P 43/00*** *(2006.01)*

(86) International application number:
**PCT/JP2008/053908**

(87) International publication number:
**WO 2008/108386 (12.09.2008 Gazette 2008/37)**

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **05.03.2007 JP 2007053675**

(71) Applicant: **Kyowa Hakko Kirin Co., Ltd.**
**Tokyo 100-8185 (JP)**

(72) Inventors:
- **SOGA, Shiro**
  **- (JP)**
- **ISHII, Toshihiko**
  **- (JP)**
- **NAKASHIMA, Takayuki**
  **- (JP)**
- **SHIOTSU, Yukimasa**
  **- (JP)**
- **AKINAGA, Shiro**
  **- (JP)**

(74) Representative: **Dossmann, Gérard et al**
**Bureau D.A. Casalonga & Josse**
**Bayerstrasse 71/73**
**80335 München (DE)**

(54) **PHARMACEUTICAL COMPOSITION**

(57) The present invention provides a pharmaceutical composition comprising a combination of an Hsp 90 family protein inhibitor and at least one compound, the said pharmaceutical composition wherein the Hsp 90 family protein inhibitor is a benozoyl compound represented by formula (I):

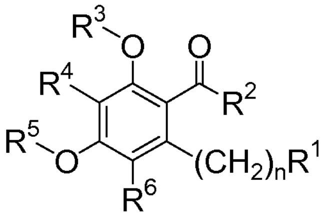

(I)

[wherein n represents an integer of 1 to 5;
$R^1$ represents substituted or unsubstituted lower alkyl, $CONR^7R^8$ (wherein $R^7$ and $R^8$, which may be the same or different, each represent a hydrogen atom, substituted or unsubstituted lower alkyl, or the like), or the like;
$R^2$ represents substituted or unsubstituted aryl, or the like;
$R^3$ and $R^5$, which may be the same or different, each represent a hydrogen atom, substituted or unsubstituted lower alkyl, or the like;
$R^4$ represents a hydrogen atom, hydroxy or halogen; and
$R^6$ represents a hydrogen atom, halogen, substituted or unsubstituted lower alkyl, or the like], or a prodrug thereof; or a pharmaceutically acceptable salt thereof, and the like.

EP 2 133 094 A1

## Description

### Technical Field

The present invention relates to a pharmaceutical composition comprising a combination of a heat shock protein 90 (hereinafter Hsp90) family protein inhibitor and at least one compound, and the like.

### Background Art

Hsp is a series of proteins expressed in cells when the cells are exposed to a stress environment such as heat shock, and are classified into families such as Hsp90, Hsp70 and Hsp60 according to their molecular weight. These proteins are also called molecular chaperones, and generally, folding, membrane transport, association, aggregation suppression of proteins, etc. are recognized as their main functions.

Hsp90 is a generic name for Hsps having a molecular weight of about 90kDa. As the Hsp90 family of eukaryotes, Hsp90α, Hsp90β, Grp94, Hsp72/TRAP1, etc. have been identified. These Hsps belonging to the Hsp90 family are hereinafter generically called Hsp90.

In recent years, it has been clarified that Hsp90 forms a complex specifically with a molecule involved in cell growth and tumorigenesis to participate in the cell cycle, cell growth, cell survival, cell immortalization, angiogenesis, metastasis and invasion. Proteins forming a complex specifically with Hsp90 are called Hsp90 client proteins. In order to maintain the function and stability of the Hsp90 client proteins in the cell, interacting with Hsp90 is considered to be necessary. Examples of known Hsp90 client proteins are steroid hormone receptors (e.g., estrogen receptor, progesterone receptor and glucocorticoid receptor), non-receptor type tyrosine kinases (e.g., Src and Lck), receptor type tyrosine kinases (e.g., EGF receptor, ErbB2 and KIT), serine-threonine kinases [e.g., Raf-1, cyclin-dependent kinase (Cdk) 4, Cdk6 and Akt], fusion proteins derived from translocation of genes (e.g., Bcr-Ab1 and NPM-ALK), telomerase and HIF-1α. It is known that by modulating the activity of Hsp90, it is possible to regulate intracellular signal transduction in which these client proteins that specifically bind to Hsp90 participate [Pharmacol. Ther., Vol. 79, p. 129-168 (1997); Biochem. Pharmacol., Vol. 56, p. 675-682 (1998); Invest. New Drugs, Vol. 17, p. 361-373 (1999)].

It has been clarified that radicicol and its derivatives bind to the ATP/ADP binding site of the N-terminus domain of Hsp90 and inhibit its activity [Cell Stress Chaperones, Vol. 3, p. 100-108 (1998); J. Med. Chem., Vol. 42, p. 260-266 (1999)]. Also, ansamycin compounds such as geldanamycin and herbimycin A and their derivatives [Cell, Vol. 89, p. 239-250 (1997); J. Natl. Cancer Inst., Vol. 92, p. 242-248 (2000)], purine derivatives (WO02/236075), pyrazole derivatives (WO03/055860), isoxazole derivatives (WO03/055860), etc. have been reported as compounds that bind to the same site of the N-terminus domain of Hsp90.

Hsp90 functions by forming a molecular complex with the above-mentioned Hsp90 client proteins together with associating molecules such as p50/Cdc37 and p23. These low-molecular compounds are considered to show various biological activities including suppression of growth of cancer cells and induction of apoptosis by binding to the ATP/ADP binding site of the N-terminus domain of Hsp90, thereby changing the construction and combination of the molecular complexes containing Hsp90, and consequently altering the function, intracellular localization or intracellular stability of Hsp90 client proteins [Invest. New Drugs, Vol. 17, p. 361-373 (1999)]. Coumarin compounds such as novobiocin are reported to show effects similar to those of the above-mentioned low-molecular compounds which bind to the N-terminus domain, by binding to the C-terminal domain (contained in amino acids 380-728) of Hsp90 [J. Natl. Cancer Inst., Vol. 92, p. 242-248 (2000)]. Geldanamycin derivatives [Invest. New Drugs, Vol. 17, p. 361-373 (1999)] and radicicol derivatives [Cancer Res., Vol. 59, p. 2931-2938 (1999); Blood, Vol. 96, p. 2284-2291 (2000); Cancer Chemother. Pharmacol., Vol. 48, p. 435-445 (2001)] are reported to show antitumor effects in animal models as well.

Hsp90 interacts with many client proteins participating in the cell cycle, cell growth, cell survival, cell immortalization, angiogenesis, metastasis and invasion, and regulates diverse phenomena associated with cancer. Therefore, it is considered that an Hsp90 family protein inhibitor shows a synergistic effect in combination with various types of antitumor agents (e.g., protein drugs, chemotherapeutic agents, hormone therapeutic agents, molecular targeted drugs, differentiation-inducing agents and antisense oligonucleotides), radiotheraphy, immunotherapy, etc. Actually, as to geldanamycin and its derivatives, there have been many reports on their combination use with antitumor agents and it is reported that they are useful as the combined agent [Current Medicinal Chemistry, Vol. 14, p. 223-232 (2007)].

For example, it is reported that when a human lung cancer-derived cell line is treated with 17-allylamino-17-demethoxygeldanamycin (17-AAG) to induce the degradation of Hsp90 client proteins involved in NF-kβ activation (e.g., RIP and IKKβ) and then treated with TNFα or TRAIL, their apoptosis-inducing activity is enhanced [Cancer Res., Vol. 66, p. 1089-1095 (2006)].

It is reported that 17-AAG, which induces the depletion of Cdk and PLK to induce the cell cycle arrest and apoptosis in a Hodgkin lymphoma-derived cell line, induces the depletion of apoptosis inhibitory proteins Akt and FLIP and so its combination use with an apoptosis-inducing agent [anti-TRAIL receptor agonist antibody (HTG-ETR1 or HTG-ETR2)] or doxorubicin enhances their effects [Clin. Cancer Res., Vol. 12, p. 584-590 (2006)].

It is reported that after in vitro treatment of a human lymphoma-derived cell line with doxorubicin, continuous use of 17-dimethylaminoethylamino-17-demethoxygeldanamycin (17-DMAG) enhances the antitumor activity of doxorubicin regardless of the status of p53 in the cells [Clin. Cancer Res., Vol. 12, p. 6547-6556 (2006)].

It is reported when a human leukemia-derived cell line is treated in vitro with 17-AAG and arsenic trioxide in combination, the antitumor activity of arsenic trioxide is enhanced because 17-AAG accelerates the degradation of Akt exerting apoptosis inhibitory effects. Also in examinations using primary cultured cells derived from acute myelogenous leukemia or chronic lymphocytic leukemia patients, similar results have been reported [Leukemia, Vol. 20, p. 610-619 (2006)].

It is reported that when a human ovarian cancer-derived cell line in which Akt is activated is treated in vitro with 17-AAG and paclitaxel simultaneously, 17-AAG enhances the antitumor activity of paclitaxel [Mol. Cancer Thr., Vol. 5, p. 1197-1208 (2006)].

It is reported that when a human solid cancer-derived cell line is treated in vitro with 17-DMAG and then with radiation, the effect of radiation is enhanced because 17-DMAG induces the degradation of a protein which attenuates radiosensitivity. It is also reported that in a model subcutaneously transplanted with DU-145, radiation after 17-AAG administration enhances its antitumor activity [Clin. Cancer Res., Vol. 10, p. 8077-8084 (2004)].

It is reported that when cell lines derived from prostatic cancer and glima are treated in vitro with 17-AAG and then with radiation, the effect of radiation is enhanced in colony assay [Clin. Cancer Res., Vol. 9, p. 3749-3755 (2003)].

It is reported that in an antitumor evaluation on a subcutaneous transplant model with a breast cancer cell line, simultaneous administration of 17-AAG and paclitaxel brings about enhancement of the antitumor activity of paclitaxel by 17-AAG [Cancer Res., Vol. 63, p. 2139-2144 (2003)].

It is reported that in vitro treatment of ErbB2-expressing cancer cells with a combination of geldanamycin and ErbB kinase inhibitor C-1033 enhances ErbB2 inhibition and thereby additively enhances antitumor activity [EMBO Journal, Vol. 21, p. 2407-2417 (2002)].

It is reported that after in vitro treatment of a human prostatic cancer-derived cell line with survivin siRNA, continuous use of 17-AAG enhances cell growth inhibition and apoptosis-inducing activity [Mol. Cancer Thr., Vol. 5, p. 179-186 (2006)].

It is reported that when primary cultured cells derived from human multiple myeloma are treated in vitro with 17-AAG and proteasome inhibitor bortezomib simultaneously, their antitumor activities are enhanced [Blood, Vol. 107, p. 1092-1100 (2006)].

It is reported that in vitro treatment of a human breast cancer-derived cell line with a combination of bortezomib and geldanamycin shows a more potent growth inhibitory activity than the treatment with either single compound [Mol. Cancer Ther., Vol. 3, p. 551-566 (2004)].

The Hsp90 family protein inhibitors used in the present invention are known (patent document Nos. 1, 2 and 3).

Patent document No. 1: W02005/000778 pamphlet
Patent document No. 2: W02005/063222 pamphlet
Patent document No. 3: WO2006/088193 pamphlet

Disclosure of the Invention

Problems to be Solved by the Invention

An object of the present invention is to provide a pharmaceutical composition comprising a combination of an Hsp90 family protein inhibitor and at least one compound, and the like.

Means for Solving the Problems

The present invention relates to the following (1) to (63).

(1) A pharmaceutical composition comprising a combination of an Hsp90 family protein inhibitor and at least one compound.
(2) A pharmaceutical composition for administering a combination of an Hsp90 family protein inhibitor and at least one compound.
(3) A pharmaceutical composition for administering an Hsp90 family protein inhibitor and at least one compound simultaneously or successively.
(4) The pharmaceutical composition according to any of the above (1) to (3), wherein the Hsp90 family protein inhibitor is a benzoyl compound represented by formula (I):

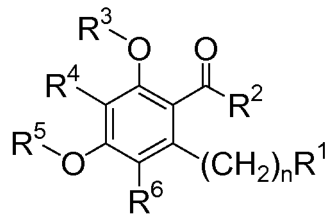

(I)

[wherein n represents an integer of 1 to 5;

$R^1$ represents substituted or unsubstituted lower alkyl, substituted or unsubstituted lower alkoxy, substituted or unsubstituted cycloalkyl, substituted or unsubstituted lower alkoxycarbonyl, substituted or unsubstituted heterocycle-alkyl, substituted or unsubstituted aryl, $CONR^7R^8$ (wherein $R^7$ and $R^8$, which may be the same or different, each represent a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, or substituted or unsubstituted aroyl, or $R^7$ and $R^8$ form a substituted or unsubstituted heterocyclic group together with the adjacent nitrogen atom thereto) or $NR^9R^{10}$ (wherein $R^9$ and $R^{10}$ have the same meanings as the above $R^7$ and $R^8$, respectively);
$R^2$ represents substituted or unsubstituted aryl or a substituted or unsubstituted aromatic heterocyclic group;
$R^3$ and $R^5$, which may be the same or different, each represent a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted aralkyl, or substituted or unsubstituted aroyl;
$R^4$ represents a hydrogen atom, hydroxy or halogen; and
$R^6$ represents a hydrogen atom, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted lower alkanoyl, substituted or unsubstituted aryloxy, substituted or unsubstituted aryl, a substituted or unsubstituted heterocyclic group, substituted or unsubstituted aralkyl, or substituted or unsubstituted heterocycle-alkyl;
provided that:

(i) when $R^3$ and $R^5$ are methyl, and $R^4$ and $R^6$ are hydrogen atoms, and

(a) when $-(CH_2)_nR^1$ is methoxycarbonylmethyl,
$R^2$ is not a group selected from the group consisting of 2,4,6-trimethoxy-5-methoxycarbonyl-3-nitrophenyl, 3-cyano-2,4,6-trimethoxyphenyl, 5-cyano-2-ethoxy-4,6-dimethoxy-3-nitrophenyl, 2,6-dimethoxyphenyl, 2-chloro-6-methoxyphenyl and 2-chloro-4,6-dimethoxy-5-methoxycarbonyl-3-nitrophenyl,
(b) when $-(CH_2)_nR^1$ is ethoxycarbonylmethyl,
$R^2$ is not 2,4,6-trimethoxy-3-methoxycarbonylphenyl,
(c) when $-(CH_2)_nR^1$ is N,N-dimethylaminomethyl,
$R^2$ is not phenyl;

(ii) when $R^3$, $R^4$, $R^5$ and $R^6$ are hydrogen atoms, and

(a) when $-(CH_2)_nR^1$ is 2-(acetoxymethyl)heptyl, 3-oxopentyl or pentyl,
$R^2$ is not 6-hydroxy-4-methoxy-3-methoxycarbonyl-2-pentylphenyl,
(b) when $-(CH_2)_nR^1$ is 3-oxopentyl,
$R^2$ is not a group selected from the group consisting of 3-benzyloxycarbonyl-6-hydroxy-4-methoxy-2-pentylphenyl and 3-carboxy-6-hydroxy-4-methoxy-2-pentylphenyl,
(c) when $-(CH_2)_nR^1$ is n-propyl,
$R^2$ is not 2,4-dihydroxy-6-[(4-hydroxy-2-oxopyran-6-yl)methyl]phenyl;

(iii) when $R^3$ and $R^4$ are hydrogen atoms, $R^5$ is methyl, $R^6$ is methoxycarbonyl, and $-(CH_2)_nR^1$ is pentyl, $R^2$ is not a group selected from the group consisting of 6-[2-(acetoxymethyl)heptyl]-2,4-dihydroxyphenyl, 2,4-dihydroxy-6-pentylphenyl and 2,4-dihydroxy-6-(3-oxopentyl)phenyl;

(iv) when $R^3$ and $R^5$ are benzyl, $R^4$ and $R^6$ are hydrogen atoms, and - $(CH_2)_nR^1$ is 3-oxopentyl, $R^2$ is not a group selected from the group consisting of 6-benzyloxy-4-methoxy-3-methoxycarbonyl-2-pentylphenyl and 6-benzyloxy-3-benzyloxycarbonyl-4-methoxy-2-pentylphenyl;
(v) when $R^3$ is benzyl, $R^4$ is a hydrogen atom, $R^5$ is methyl, - $(CH_2)_nR^1$ is pentyl, and $R^6$ is methoxycarbonyl or benzyloxycarbonyl,
$R^2$ is not 2,4-bis(benzyloxy)-6-(3-oxopentyl)phenyl;
(vi) when $R^3$ and $R^4$ are hydrogen atoms, $R^5$ is methyl, -$(CH_2)_nR^1$ is pentyl, and $R^6$ is carboxy or benzyloxycarbonyl,
$R^2$ is not 2,4-dihydroxy-6-(3-oxopentyl)phenyl; and
(vii) when $R^3$, $R^4$ and $R^6$ are hydrogen atoms, $R^5$ is n-propyl, and - $(CH_2)_nR^1$ is 5-(1,1-dimethylpropyl)-4-(2-hydrobenzotriazol-2-yl)-2-hydroxyphenylmethyl,
$R^2$ is not phenyl],

or a prodrug thereof; or a pharmaceutically acceptable salt thereof.

(5) The pharmaceutical composition according to the above (4), wherein $R^2$ is a substituted or unsubstituted aromatic heterocyclic group, aryl substituted with 1 to 3 substituents or aryl.
(6) The pharmaceutical composition according to the above (4), wherein $R^2$ is aryl substituted with 1 to 3 substituents or aryl.
(7) The pharmaceutical composition according to the above (4), wherein $R^2$ is phenyl substituted with 1 to 3 substituents or phenyl.
(8) The pharmaceutical composition according to the above (4), wherein $R^2$ is a substituted or unsubstituted aromatic heterocyclic group.
(9) The pharmaceutical composition according to any of the above (4) to (8), wherein $R^3$ and $R^5$, which may be the same or different, each are a hydrogen atom, substituted or unsubstituted lower alkyl, substituted or unsubstituted lower alkanoyl, substituted or unsubstituted aroyl, or substituted or unsubstituted lower alkenyl.
(10) The pharmaceutical composition according to any of the above (4) to (8), wherein $R^3$, $R^4$ and $R^5$ are hydrogen atoms.
(11) The pharmaceutical composition according to any of the above (4) to (10), wherein $R^1$ is $CONR^7R^8$ (wherein $R^7$ and $R^8$ have the same meanings as defined above, respectively).
(12) The pharmaceutical composition according to any of the above (4) to (10), wherein $R^1$ is $CONR^{7a}R^{8a}$ (wherein $R^{7a}$ and $R^{8a}$, which may be the same or different, each represent a hydrogen atom, substituted or unsubstituted lower alkyl, or substituted or unsubstituted heterocycle-alkyl).
(13) The pharmaceutical composition according to any of the above (4) to (10), wherein $R^1$ is $CONR^{7b}R^{8b}$ (wherein $R^{7b}$ and $R^{8b}$ form a substituted or unsubstituted heterocyclic group together with the adjacent nitrogen atom thereto).
(14) The pharmaceutical composition according to any of the above (4) to (10), wherein $R^1$ is substituted or unsubstituted lower alkoxy.
(15) The pharmaceutical composition according to any of the above (4) to (14), wherein $R^6$ is a hydrogen atom, lower alkyl, halogen or aryl.
(16) The pharmaceutical composition according to any of the above (4) to (14), wherein $R^6$ is lower alkyl.
(17) The pharmaceutical composition according to any of the above (4) to (14), wherein $R^6$ is ethyl.
(18) The pharmaceutical composition according to any of the above (1) to (3), wherein the Hsp90 family protein inhibitor is a benzoyl compound represented by formula (IA):

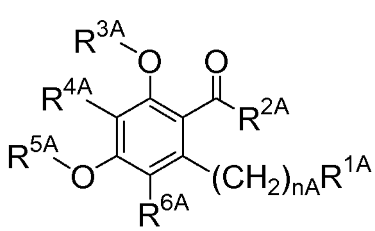
(IA)

[wherein nA represents an integer of 0 to 10;

$R^{1A}$ represents a hydrogen atom, hydroxy, cyano, carboxy, nitro, halogen, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted lower alkanoyloxy, substituted or unsubstituted heterocycle-alkyl, substituted or unsubstituted aryl, substituted or unsubstituted arylsulfonyl, a substituted or unsubstituted heterocyclic group, $CONR^7R^8$ (wherein $R^7$ and $R^8$ have the same meanings as defined above, respectively) or $NR^9R^{10}$ (wherein $R^9$ and $R^{10}$ have the same meanings as defined above, respectively);

$R^{2A}$ represents substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group;

$R^{3A}$ and $R^{5A}$, which may be the same or different, each represent a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted aralkyl, or substituted or unsubstituted aroyl; and

$R^{4A}$ and $R^{6A}$, which may be the same or different, each represent a hydrogen atom, hydroxy, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted lower alkanoyl, substituted or unsubstituted aryloxy, substituted or unsubstituted aryl, a substituted or unsubstituted heterocyclic group, substituted or unsubstituted aralkyl, or substituted or unsubstituted heterocycle-alkyl], or a prodrug thereof; or a pharmaceutically acceptable salt thereof.

(19) The pharmaceutical composition according to any of the above (1) to (3), wherein the Hsp90 family protein inhibitor is a benzene derivative represented by formula (II):

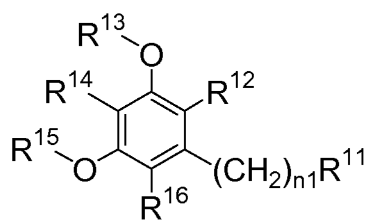

(II)

{wherein n1 represents an integer of 0 to 10;

$R^{11}$ represents a hydrogen atom, hydroxy, cyano, carboxy, nitro, halogen, substituted or unsubstituted lower alkyl, substituted or unsubstituted lower alkenyl, substituted or unsubstituted lower alkynyl, substituted or unsubstituted cycloalkyl, substituted or unsubstituted lower alkoxycarbonyl, substituted or unsubstituted aroyl, substituted or unsubstituted lower alkanoyl, substituted or unsubstituted heterocycle-alkyl, substituted or unsubstituted aryl, substituted or unsubstituted aralkyl, substituted or unsubstituted arylsulfonyl, a substituted or unsubstituted heterocyclic group, $CONR^{17}R^{18}$ (wherein $R^{17}$ and $R^{18}$, which may be the same or different, each represent a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, or substituted or unsubstituted aroyl, or $R^{17}$ and $R^{18}$ form a substituted or unsubstituted heterocyclic group together with the adjacent nitrogen atom thereto), $NR^{19}R^{20}$ [wherein $R^{19}$ and $R^{20}$, which may be the same or different, each represent a hydrogen atom, substituted or unsubstituted lower alkylsulfonyl, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, substituted or unsubstituted aroyl or $CONR^{21}R^{22}$ (wherein $R^{21}$ and $R^{22}$ have the same meanings as the above $R^{17}$ and $R^{18}$, respectively), or $R^{19}$ and $R^{20}$ form a substituted or unsubstituted heterocyclic group together with the adjacent nitrogen atom thereto], or $OR^{23}$ (wherein $R^{23}$ represents substituted or unsubstituted lower alkyl, substituted or unsubstituted lower alkenyl, substituted or unsubstituted lower alkanoyl, substituted or unsubstituted aryl, a substituted or unsubstituted heterocyclic group, substituted or unsubstituted aralkyl, or substituted or unsubstituted heterocycle-alkyl); $R^{12}$ represents substituted or unsubstituted lower alkyl, substituted or unsubstituted lower alkenyl, substituted or unsubstituted lower alkynyl, substituted or unsubstituted aryl, or a substituted or unsubstituted heterocyclic group (excluding substituted or unsubstituted pyrazolyl); $R^{13}$ and $R^{15}$, which may be the same or different, each represent a hydrogen atom, substituted or unsubstituted lower alkyl, substituted or unsubstituted lower alkenyl, substituted or unsubstituted

lower alkanoyl, substituted or unsubstituted cycloalkyl, substituted or unsubstituted lower alkylsulfonyl, substituted or unsubstituted arylsulfonyl, carbamoyl, sulfamoyl, substituted or unsubstituted lower alkylaminocarbonyl, substituted or unsubstituted di-lower alkylaminocarbonyl, substituted or unsubstituted lower alkoxycarbonyl, substituted or unsubstituted heterocycle-carbonyl, substituted or unsubstituted aralkyl, or substituted or unsubstituted aroyl; and

$R^{14}$ and $R^{16}$, which may be the same or different, each represent a hydrogen atom, hydroxy, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted aryloxy, substituted or unsubstituted aryl, a substituted or unsubstituted heterocyclic group (excluding substituted or unsubstituted pyrazolyl), substituted or unsubstituted lower alkanoyl, substituted or unsubstituted aralkyl, or substituted or unsubstituted heterocycle-alkyl},

or a prodrug thereof; or a pharmaceutically acceptable salt thereof.

(20) The pharmaceutical composition according to the above (19), wherein $R^{11}$ is a hydrogen atom, hydroxy, cyano, carboxy, nitro, halogen, substituted or unsubstituted lower alkyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted lower alkanoyloxy, substituted or unsubstituted heterocycle-alkyl, substituted or unsubstituted aryl, substituted or unsubstituted arylsulfonyl, $CONR^{17}R^{18}$ (wherein $R^{17}$ and $R^{18}$ have the same meanings as defined above, respectively), or $NR^{19}R^{20}$ (wherein $R^{19}$ and $R^{20}$ have the same meanings as defined above, respectively).

(21) The pharmaceutical composition according to the above (19), wherein $R^{11}$ is substituted or unsubstituted lower alkyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted heterocycle-alkyl, substituted or unsubstituted aryl, $CONR^{17}R^{18}$ (wherein $R^{17}$ and $R^{18}$ have the same meanings as defined above, respectively), or $NR^{19}R^{20}$ (wherein $R^{19}$ and $R^{20}$ have the same meanings as defined above, respectively).

(22) The pharmaceutical composition according to any of the above (19) to (21), wherein $R^{12}$ is substituted or unsubstituted aryl or a substituted or unsubstituted aromatic heterocyclic group.

(23) The pharmaceutical composition according to any of the above (19) to (21), wherein $R^{12}$ is substituted or unsubstituted aryl.

(24) The pharmaceutical composition according to any of the above (19) to (21), wherein $R^{12}$ is substituted or unsubstituted phenyl.

(25) The pharmaceutical composition according to any of the above (19) to (21), wherein $R^{12}$ is substituted or unsubstituted furyl.

(26) The pharmaceutical composition according to any of the above (19) to (25), wherein $R^{14}$ is a hydrogen atom, hydroxy or halogen.

(27) The pharmaceutical composition according to any of the above (19) to (26), wherein $R^{13}$ and $R^{15}$, which may be the same or different, each are a hydrogen atom, substituted or unsubstituted lower alkyl, substituted or unsubstituted lower alkenyl, substituted or unsubstituted lower alkanoyl, substituted or unsubstituted aroyl, substituted or unsubstituted lower alkylaminocarbonyl, substituted or unsubstituted di-lower alkylaminocarbonyl, substituted or unsubstituted lower alkoxycarbonyl, or substituted or unsubstituted heterocycle-carbonyl.

(28) The pharmaceutical composition according to any of the above (19) to (25), wherein $R^{13}$, $R^{14}$ and $R^{15}$ are hydrogen atoms.

(29) The pharmaceutical composition according to any of the above (1) to (28), wherein the target disease is cancer.

(30) The pharmaceutical composition according to the above (29), wherein the cancer is cancer derived from hematopoietic tumor, breast cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma, or cancer derived from brain tumor.

(31) The pharmaceutical composition according to the above (29), wherein the cancer is leukemia, myeloma or lymphoma.

(32) The pharmaceutical composition according to the above (29), wherein the cancer is acute myeloid leukemia.

(33) The pharmaceutical composition according to the above (29), wherein the cancer is multiple myeloma.

(34) The pharmaceutical composition according to the above (29), wherein the cancer is solid cancer.

(35) The pharmaceutical composition according to the above (34), wherein the solid cancer is breast cancer.

(36) The pharmaceutical composition according to the above (34), wherein the solid cancer is lung cancer.

(37) The pharmaceutical composition according to any of the above (3) to (36), wherein the compound to be administered in combination, simultaneously or successively, with the Hsp90 family protein inhibitor is a protein or a

low-molecular compound.

(38) The pharmaceutical composition according to the above (37), wherein the compound to be combined with the Hsp90 family protein inhibitor is a protein and the protein is an antibody.

(39) The pharmaceutical composition according to the above (38), wherein the antibody is an anti-ErbB2 antibody.

(40) The pharmaceutical composition according to the above (38), wherein the antibody is trastuzumab.

(41) The pharmaceutical composition according to the above (37), wherein the compound to be combined with the Hsp90 family protein inhibitor is a low-molecular compound and the low-molecular compound is a chemotherapeutic agent or a molecular targeted drug.

(42) The pharmaceutical composition according to the above (41), wherein the low-molecular compound is a chemotherapeutic agent and the chemotherapeutic agent is melphalan or paclitaxel.

(43) The pharmaceutical composition according to the above (41), wherein the low-molecular compound is a molecular targeted drug and the molecular targeted drug is a kinase inhibitor.

(44) The pharmaceutical composition according to the above (43), wherein the kinase inhibitor is gefitinib.

(45) The pharmaceutical composition according to the above (43), wherein the kinase inhibitor is a fms-like tyrosine kinase 3 (Flt-3) inhibitor.

(46) The pharmaceutical composition according to the above (43), wherein the kinase inhibitor is an Aurora inhibitor, an Abelson kinase (Abl kinase) inhibitor, a vascular endothelial growth factor receptor (VEGFR) inhibitor, a fibroblast growth factor receptor (FGFR) inhibitor, a platelet derived growth factor receptor (PDGFR) inhibitor or an ephrin inhibitor.

(47) The pharmaceutical composition according to the above (41), wherein the low-molecular compound is a molecular targeted drug and the molecular targeted drug is a proteasome inhibitor.

(48) The pharmaceutical composition according to the above (47), wherein the proteasome inhibitor is bortezomib.

(49) A method of treating cancer, which comprises the step of administering an Hsp90 family protein inhibitor and at least one compound simultaneously or separately with an interval.

(50) The method of treating cancer according to the above (49), wherein the Hsp90 family protein inhibitor is a benzoyl compound represented by formula (I):

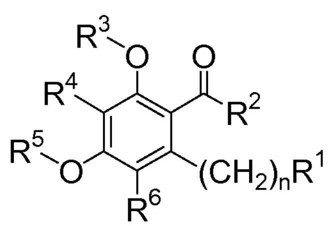

(I)

(wherein n, $R^1$, $R^2$, $R^3$, $R^4$, $R^5$ and $R^6$ have the same meanings as defined above, respectively), or a prodrug thereof; or a pharmaceutically acceptable salt thereof.

(51) The method of treating cancer according to the above (49), wherein the Hsp90 family protein inhibitor is a benzoyl compound represented by formula (IA):

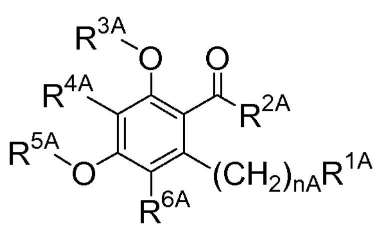

(IA)

(wherein nA, $R^{1A}$, $R^{2A}$, $R^{3A}$, $R^{4A}$, $R^{5A}$ and $R^{6A}$ have the same meanings as defined above, respectively), or a prodrug thereof; or a pharmaceutically acceptable salt thereof.

(52) The method of treating cancer according to the above (49), wherein the Hsp90 family protein inhibitor is a benzene derivative represented by formula (II):

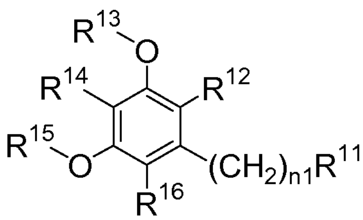

(II)

(wherein n1, $R^{11}$, $R^{12}$, $R^{13}$, $R^{14}$, $R^{15}$ and $R^{16}$ have the same meanings as defined above, respectively), or a prodrug thereof; or a pharmaceutically acceptable salt thereof.

(53) Use of an Hsp90 family protein inhibitor and at least one compound for the manufacture of an anticancer agent.

(54) The use according to the above (53), wherein the Hsp90 family protein inhibitor is a benzoyl compound represented by formula (I):

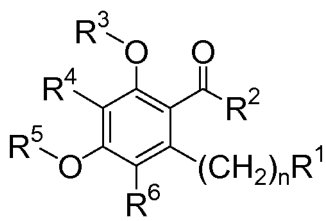

(I)

(wherein n, $R^1$, $R^2$, $R^3$, $R^4$, $R^5$ and $R^6$ have the same meanings as defined above, respectively), or a prodrug thereof; or a pharmaceutically acceptable salt thereof.

(55) The use according to the above (53), wherein the Hsp90 family protein inhibitor is a benzoyl compound represented by formula (IA):

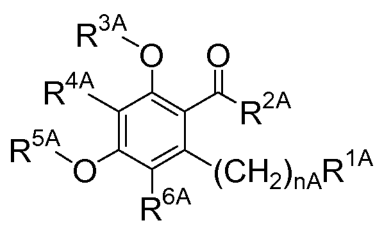

(IA)

(wherein nA, $R^{1A}$, $R^{2A}$, $R^{3A}$, $R^{4A}$, $R^{5A}$ and $R^{6A}$ have the same meanings as defined above, respectively), or a prodrug thereof; or a pharmaceutically acceptable salt thereof.

(56) The use according to the above (53), wherein the Hsp90 family protein inhibitor is a benzene derivative represented by formula (II):

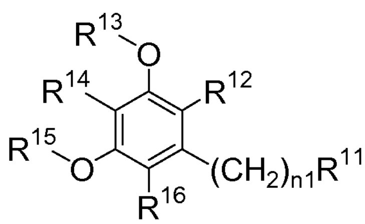

(II)

(wherein n1, $R^{11}$, $R^{12}$, $R^{13}$, $R^{14}$, $R^{15}$ and $R^{16}$ have the same meanings as defined above, respectively), or a prodrug thereof; or a pharmaceutically acceptable salt thereof.

(57) A kit which comprises a first component comprising an Hsp90 family protein inhibitor and a second component

comprising an antitumor agent.

(58) The kit according to the above (57), wherein the Hsp90 family protein inhibitor is the benzoyl compound described in any of the above (4) to (18), or a prodrug thereof; or a pharmaceutically acceptable salt thereof.

(59) The kit according to the above (57), wherein the Hsp90 family protein inhibitor is the benzene derivative described in any of the above (19) to (28), or a prodrug thereof; or a pharmaceutically acceptable salt thereof.

(60) An antitumor agent for administering an Hsp90 family protein inhibitor and at least one compound as active ingredients simultaneously or successively.

(61) The antitumor agent according to the above (60), wherein the Hsp90 family protein inhibitor is the benzoyl compound described in any of the above (4) to (18), or a prodrug thereof; or a pharmaceutically acceptable salt thereof.

(62) The antitumor agent according to the above (60), wherein the Hsp90 family protein inhibitor is the benzene derivative described in any of the above (19) to (28), or a prodrug thereof; or a pharmaceutically acceptable salt thereof.

(63) A method of treating cancer, which comprises the step of applying radiation before or after administering an Hsp90 family protein inhibitor.

## Effect of the Invention

The present invention provides a pharmaceutical composition comprising a combination of an Hsp90 family protein inhibitor and at least one compound, and the like.

## Brief Description of the Drawings

Fig. 1 shows the antitumor effect of a test compound combined with gefitinib in a mouse model transplanted with human lung cancer NCI-H596 cells. The ordinate indicates the ratio of change in tumor volume (V/V0) based on the tumor volume at day 0 (V0), and the abscissa indicates days. Solid diamonds denotes the growth inhibitory effect of administration of neither the test compound nor gefitinib; solid circles, administration of the test compound; solid triangles, administration of gefitinib; and crosses, combined administration of the test compound and gefitinib.

Fig. 2 shows the antitumor effect of a test compound combined with paclitaxel in a mouse model transplanted with human breast cancer KPL-4 cells.

The ordinate indicates the ratio of change in tumor volume (V/V0) based on the tumor volume at day 0 (V0), and the abscissa indicates days. Solid diamonds denotes the growth inhibitory effect of administration of neither the test compound nor paclitaxel; solid circles, administration of the test compound; solid triangles, administration of paclitaxel; and crosses, combined administration of the test compound and paclitaxel.

Fig. 3 shows the antitumor effect of a test compound combined with trastuzumab in a mouse model transplanted with human breast cancer KPL-4 cells.

The ordinate indicates the ratio of change in tumor volume (V/V0) based on the tumor volume at day 0 (V0), and the abscissa indicates days. Solid diamonds denotes the growth inhibitory effect of administration of neither the test compound nor trastuzumab; solid circles, administration of the test compound; solid triangles, administration of trastuzumab; and crosses, combined administration of the test compound and trastuzumab.

Fig. 4 shows the antitumor effect of a test compound combined with bortezomib in a mouse model transplanted with human multiple myeloma NCI-H929 cells.

The ordinate indicates the ratio of change in tumor volume (V/V0) based on the tumor volume at day 0 (V0), and the abscissa indicates days. Solid diamonds denotes the growth inhibitory effect of administration of neither the test compound nor bortezomib; solid circles, administration of the test compound; solid triangles, administration of bortezomib; and crosses, combined administration of the test compound and bortezomib.

Fig. 5 shows the antitumor effect of a test compound combined with melphalan in a mouse model transplanted with human multiple myeloma NCI-H929 cells.

The ordinate indicates the ratio of change in tumor volume (V/V0) based on the tumor volume at day 0 (V0), and the abscissa indicates days. Solid diamonds denotes the growth inhibitory effect of administration of neither the test compound nor melphalan; solid circles, administration of the test compound; solid triangles, administration of melphalan; and crosses, combined administration of the test compound and melphalan.

## Best Modes for Carrying Out the Invention

Hereinafter, the compounds represented by general formulae (I), (IA) and (II) are referred to as Compounds (I), (IA), (II) and the same applies to compounds of other formula numbers.

In the definitions of the groups in general formula (I), (IA) and (II):

Examples of the lower alkyl and the lower alkyl moieties of the lower alkoxy, lower alkoxycarbonyl, lower alkylaminocarbonyl di-lower alkylaminocarbonyl, lower alkylsulfonyl, lower alkylamino and di-lower alkylamino include straight-chain or branched alkyl having 1 to 8 carbon atoms, such as methyl, ethyl, propyl, isopropyl, butyl, sec-butyl, tert-butyl, pentyl, isopentyl, neopentyl, hexyl, heptyl and octyl. The two lower alkyl moieties of the di-lower alkylamino and di-lower alkylaminocarbonyl may be the same or different.

Examples of the lower alkenyl include straight-chain or branched alkenyl having 2 to 8 carbon atoms, such as vinyl, allyl, 1-propenyl, methacryl, crotyl, 1-butenyl, 3-butenyl, 2-pentenyl, 4-pentenyl, 2-hexenyl, 5-hexenyl, 2-heptenyl and 2-octenyl.

Examples of the lower alkynyl include straight-chain or branched alkynyl having 2 to 8 carbon atoms, such as ethynyl, propynyl, butynyl, pentynyl, hexynyl, heptynyl and octynyl.

Examples of the lower alkanoyl and the lower alkanoyl moiety of the lower alkanoyloxy include straight-chain or branched alkanoyl having 1 to 7 carbon atoms, such as formyl, acetyl, propionyl, butyryl, isobutyryl, valeryl, isovaleryl, pivaloyl, hexanoyl and heptanoyl.

Examples of the cycloalkyl include cycloalkyl having 3 to 8 carbon atoms, such as cyclopropyl, cyclobutyl, cyclopentyl, cyclohexyl, cycloheptyl and cyclooctyl.

Examples of the aryl and the aryl moiety of the arylsulfonyl, aryloxy and aroyl include monocyclic, bicyclic or tricyclic aryl having 6 to 14 carbon atoms, such as phenyl, indenyl, naphthyl and anthryl.

Examples of the aralkyl include aralkyl having 7 to 15 carbon atoms, such as benzyl, phenethyl, benzhydryl and naphthylmethyl.

Examples of the aromatic heterocyclic group include 5- or 6-membered monocyclic aromatic heterocyclic groups containing at least one atom selected from a nitrogen atom, an oxygen atom and a sulfur atom, and bicyclic or tricyclic condensed aromatic heterocyclic groups containing at least one atom selected from a nitrogen atom, an oxygen atom and a sulfur atom in which 3- to 8-membered rings are condensed, such as pyridyl, pyrazinyl, pyrimidinyl, pyridazinyl, quinolinyl, isoquinolinyl, phthalazinyl, quinazolinyl, quinoxalinyl, naphthyridinyl, cinnolinyl, pyrrolyl, pyrazolyl, imidazolyl, triazolyl, tetrazolyl, thienyl, furyl, thiazolyl, oxazolyl, indolyl, indazolyl, benzimidazolyl, benzotriazolyl, benzothiazolyl, benzoxazolyl, purinyl and benzodioxolanyl.

Examples of the heterocyclic group and the heterocyclic group moiety of the heterocycle-carbonyl and heterocycle-alkyl include groups described in the above definition of the aromatic heterocyclic group and also aliphatic heterocyclic groups. Examples of the aliphatic heterocyclic group include 5- or 6-membered monocyclic aliphatic heterocyclic groups containing at least one atom selected from a nitrogen atom, an oxygen atom and a sulfur atom, and bicyclic or tricyclic condensed aliphatic heterocyclic groups containing at least one atom selected from a nitrogen atom, an oxygen atom and a sulfur atom in which 3- to 8-membered rings are condensed, such as pyrrolidinyl, piperidino, piperazinyl, piperazinyl, morpholino, morpholinyl, thiomorpholino, thiomorpholinyl, homopiperidino, homopiperazinyl, homopiperazinyl, tetrahydropyridinyl, tetrahydroquinolinyl, tetrahydroisoquinolinyl, tetrahydrofuranyl, tetrahydropyranyl, dihydrobenzofuranyl, oxopiperazinyl and 2-oxopyrrolidinyl.

Examples of the heterocyclic group formed together with the adjacent nitrogen atom include 5- or 6-membered monocyclic heterocyclic groups containing at least one nitrogen atom (the monocyclic heterocyclic groups may also contain another nitrogen atom, an oxygen atom or a sulfur atom), and bicyclic or tricyclic condensed heterocyclic groups containing at least one nitrogen atom in which 3- to 8-membered rings are condensed (the condensed heterocyclic groups may also contain another nitrogen atom, an oxygen atom or a sulfur atom), such as pyrrolidinyl, piperidino, piperazinyl, morpholino, thiomorpholino, homopiperidino, homopiperazinyl, tetrahydropyridinyl, tetrahydroquinolinyl, tetrahydroisoquinolinyl, oxopiperazinyl and 2-oxopyrrolidinyl.

The alkylene moiety of the heterocycle-alkyl has the same meaning as a group produced by removing one hydrogen atom from the lower alkyl defined above.

The halogen means fluorine, chlorine, bromine and iodine atoms.

Examples of the substituents (A) in the substituted lower alkyl, the substituted lower alkoxy, the substituted lower alkoxycarbonyl, the substituted di-lower alkylaminocarbonyl, the substituted lower alkylaminocarbonyl, substituted lower alkylsulfonyl and the substituted lower alkenyl, and the substituted lower alkynyl include 1 to 3 substituents which are the same or different, such as hydroxy, oxo, cyano, nitro, carboxy, amino, halogen, substituted or unsubstituted lower alkoxy, cycloalkyl, lower alkanoyl, lower alkoxycarbonyl, lower alkylamino and di-lower alkylamino. The position (s) to be substituted with the substituent(s) is/are not particularly limited. Here, the halogen, the lower alkoxy, the cycloalkyl, the lower alkanoyl, the lower alkoxycarbonyl, the lower alkylamino and the di-lower alkylamino each have the same meanings as defined above. Examples of the substituents in the substituted lower alkoxy include 1 to 3 substituents which are the same or different, such as hydroxy and halogen, and the halogen has the same meaning as defined above.

Examples of the substituents (B) in the substituted lower alkanoyl, the substituted lower alkanoyloxy, the substituted cycloalkyl, the substituted aryl, the substituted arylsulfonyl, the substituted aryloxy, the substituted aralkyl, the substituted aroyl, the substituted heterocycle-alkyl, the substituted heterocyclic group, the substituted heterocycle-carbonyl, the substituted aromatic heterocyclic group and the substituted heterocyclic group formed together with the adjacent nitrogen atom include 1 to 3 substituents which are the same or different, such as hydroxy, halogen, nitro, cyano, amino, carboxy, carbamoyl, substituted or unsubstituted lower alkyl, substituted or unsubstituted lower alkoxy, aralkyloxy, lower alkylsulfonyl, lower alkylsulfanyl, cycloalkyl, lower alkoxycarbonyl, lower alkylamino, di-lower alkylamino, lower alkanoyl, a heterocyclic group, substituted or unsubstituted aryl, substituted or unsubstituted heterocycle-alkyloxy, and substituted or unsubstituted heterocycle-carbonylalkyloxy. The position(s) to be substituted with substituent (s) is/are not particularly limited. Here, the halogen, the lower alkyl, the lower alkoxy, the cycloalkyl, the lower alkoxycarbonyl, the lower alkylamino, the di-lower alkylamino, the lower alkanoyl, the heterocyclic group and the aryl each have the same meanings as defined above; the lower alkyl moiety of the lower alkylsulfonyl and lower alkylsulfanyl has the same meaning as the above-described lower alkyl; the aralkyl moiety of the aralkyloxy has the same meaning as the above-described aralkyl; and the heterocyclic group moiety and the alkylene of the heterocycle-alkyloxy and heterocycle-carbonylalkyloxy, respectively, have the same meanings as the above-described heterocyclic group and the group produced by removing a hydrogen atom from the above-described lower alkyl. Examples of the substituents in the substituted lower alkyl, the substituted lower alkoxy and the substituted aryl include 1 to 3 substituents which are the same or different, such as hydroxy, halogen, lower alkoxy, cyano, lower alkylamino and di-lower alkylamino. Herein, the halogen, the lower alkoxy, the lower alkylamino and the di-lower alkylamino each have the same meanings as defined above. Examples of the substituents in the substituted heterocycle-alkyloxy and the substituted heterocycle-carbonylalkyloxy include 1 to 3 substituents which are the same or different, such as hydroxy, halogen, lower alkyl, lower alkoxy and a heterocyclic group. Herein, the halogen, the lower alkyl, the lower alkoxy and the heterocyclic group each have the same meanings as defined above.

The prodrugs of Compounds (I), (IA) and (II) include compounds which are converted in vivo, for example, by various mechanisms such as hydrolysis in blood to form Compounds (I), (IA) and (II) of the present invention, and the like. Such compounds can be specified by techniques well known in the art (e.g. J. Med. Chem., 1997, Vol. 40, p. 2011-2016; Drug Dev. Res., 1995, Vol. 34, p. 220-230; Advances in Drug Res., 1984, Vol. 13, p. 224-331; Bundgaard, Design of Prodrugs, 1985, Elsevier Press and the like).

Specifically, when Compounds (I), (IA) and (II) have carboxy in their structure, examples of prodrugs of Compounds (I), (IA) and (II) include compounds in which the hydrogen atom of said carboxy is substituted with a group selected from lower alkyl, lower alkanoyloxyalkyl [e.g. lower alkanoyloxymethyl, 1-(lower alkanoyloxy)ethyl and 1-methyl-1-(lower alkanoyloxy)ethyl], lower alkoxycarbonyloxyalkyl [e.g. lower alkoxycarbonyloxymethyl, 1-(lower alkoxycarbonyloxy)ethyl, and 1-methyl-1-(lower alkoxycarbonyloxy)ethyl], N-(lower alkoxycarbonyl)aminoalkyl {e.g. N-(lower alkoxycarbonyl)aminomethyl and 1-[N-(lower alkoxycarbonyl)amino]ethyl}, 3-phthalidyl, 4-crotonolactonyl, γ-butyrolacton-4-yl, di-lower alkylaminoalkyl, carbamoylalkyl, di-lower alkylcarbamoylalkyl, piperidinoalkyl, pyrrolidinoalkyl, morpholinoalkyl and the like.

Also, when Compounds (I), (IA) and (II) have alcoholic hydroxy in its structure, examples of prodrugs of Compounds (I), (IA) and (II) include compounds in which the hydrogen atom of said hydroxy is substituted with a group selected from lower alkanoyloxyalkyl, 1-(lower alkanoyloxy)ethyl, 1-methyl-1-(lower alkanoyloxy)ethyl, lower alkoxycarbonyloxyalkyl, N-(lower alkoxycarbonyl)aminoalkyl, succinoyl, lower alkanoyl, α-amino lower alkanoyl and the like.

Also, when Compounds (I), (IA) and (II) have amino in their structure, examples of prodrugs of Compounds (I), (IA) and (II) include compounds in which one or two hydrogen atoms of said amino are substituted with a group selected from lower alkylcarbonyl, lower alkoxycarbonyl, lower alkylcarbamoyl, di-lower alkylcarbamoyl and the like.

The lower alkyl and the lower alkyl moiety of the above-described lower alkoxycarbonyloxyalkyl, lower alkoxycarbonyloxymethyl, 1-(lower alkoxycarbonyloxy)ethyl, 1-methyl-1-(lower alkoxycarbonyloxy)ethyl, N-(lower alkoxycarbonyl)aminoalkyl, N-(lower alkoxycarbonyl)aminomethyl, 1-[N-(lower alkoxycarbonyl)amino]ethyl, di-lower alkylaminoalkyl, di-lower alkylcarbamoylalkyl, lower alkylcarbonyl, lower alkoxycarbonyl, lower alkylcarbamoyl and di-lower alkylcarbamoyl has the same meaning as the above-described lower alkyl. The two lower alkyl moieties of the di-lower alkylaminoalkyl, di-lower alkylcarbamoylalkyl and di-lower alkylcarbamoyl may be the same or different.

Also, the lower alkanoyl moiety of the above-described lower alkanoyloxyalkyl, lower alkanoyloxymethyl, 1-(lower alkanoyloxy)ethyl, 1-methyl-1-(lower alkanoyloxy)ethyl, lower alkanoyl and α-amino lower alkanoyl has the same meaning as the above-described lower alkanoyl.

Also, the alkylene moiety of the above-described lower alkanoyloxyalkyl, lower alkoxycarbonyloxyalkyl, N-(lower alkoxycarbonyl)aminoalkyl, di-lower alkylaminoalkyl, carbamoylalkyl, di-lower alkylcarbamoylalkyl, piperidinoalkyl, pyrrolidinoalkyl and morpholinoalkyl has the same meaning as the group produced by removing a hydrogen atom from the above-described lower alkyl.

These prodrugs of Compound (I) can be prepared from Compound (I) according to, for example, the methods described in T.W. Greene, Protective Groups in Organic Synthesis, third edition, John Wiley & Sons Inc. (1999), or methods similar thereto.

The pharmaceutically acceptable salts of Compounds (I), (IA) and (II) or prodrugs thereof include pharmaceutically acceptable acid addition salts, metal salts, ammonium salts, organic amine addition salts and amino acid addition

salts.

Examples of the pharmaceutically acceptable acid addition salts of Compounds (I), (IA) and (II) or prodrugs thereof include inorganic acid addition salts such as hydrochloride, sulfate, nitrate and phosphate, and organic acid addition salts such as acetate, maleate, fumarate and citrate. Examples of the pharmaceutically acceptable metal salts include alkali metal salts such as sodium salt and potassium salt, alkaline earth metal salts such as magnesium salt and calcium salt, aluminum salt, and zinc salt. Examples of the pharmaceutically acceptable ammonium salts include ammonium and tetramethylammonium. Examples of the pharmaceutically acceptable organic amine addition salts include an addition salt of morpholine or piperidine. Examples of the pharmaceutically acceptable amino acid addition salts include an addition salt of glycine, phenylalanine, lysine, aspartic acid or glutamic acid.

The term "inhibition of Hsp90 family protein" refers to inhibition of the binding of Hsp90 family protein to a protein to which Hsp90 family protein binds (Hsp90 client protein).

Examples of Hsp90 family proteins include Hsp90α protein, Hsp90β protein, grp94 and hsp75/TRAP1.

The proteins to which Hsp90 family proteins bind include any proteins to which Hsp90 family proteins bind, for example, EGFR, Erb-B2, Bcr-Ab1, src, raf-1, AKT, Flt-3, PLK, Wee1, FAK, cMET, hTERT, HIF1-α, mutant p53, estrogen receptors and androgen receptors (Expert Opinion on Biological Therapy, 2002, Vol. 2, p. 3-24).

Of pharmaceutical compounds of the present invention, compounds which can be used by binding together with the Hsp90 family proteins inhibitor include antitumor agents and proteins or low-molecular compounds besides antitumor agents. Examples of antitumor agents include compounds used for the treatment of cancer such as protein drugs, chemotherapeutic agents, hormone therapeutic agents, molecular targeted drugs, differentiation-inducing agents, bone resorption inhibitors, nucleic acid drugs (siRNA and antisense oligonucleotides). Also, cancers can be treated by radiation (radiotheraphy) prior to or after the administration of Hsp90 family protein inhibitors.

Examples of the radiation for radiotherapy include negatron, positive electron, proton, fast neutron, negative pion, heavy ion, charged particle, x-ray, γ-ray, radiowave, infrared ray, ultraviolet ray, optical wavelength and the like.

Examples of the protein drugs include, cytokine, antibody and the like.

Examples of the cytokine include, interleukin-2(IL-2), IFN-α, IFN-γ, GM-CSF, G-CSF, TNF-α, IL-1β and the like.

Examples of the antibody include anti-EGFR antibody [cetuximab (Erbitux)], anti-ErbB2antibody [trastuzumab (Herceptin)], anti-VEGF antibody [bevacizumab (Avastin)], anti-CD20 antibody [rituximab (Rituxan), anti-CD33 antibody [gemtuzumab ozogamicin (Mylotarg)], anti-CD52 antibody [alemtuzumab(Campath)], anti-TRAIL antibody and the like.

Examples of the chemotherapeutic agents include tublin acting agent, DNA acting agent, antimetabolite and the like.

Examples of the hormone therapeutic agents include anti-androgen agent, anti-estrogen agent, androgen preparation, estrogen preparation, LH-RH agonist (chemical castration drug), progestin, aromatase inhibitor, steroid sulfatase inhibitor, and the like.

Examples of the molecular targeted drug include Bcr-Abl inhibitor, EGFR inhibitor, JAK inhibitor, multikinase inhibitor, kinesine Eg5 inhibitor, Flt-3 inhibitor, mTOR inhibitor, proteasome inhibitor, HDAC inhibitor, DNA methylation inhibitor, farnesyltransferase inhibitor, Bcl-2 inhibitor, Aurora inhibitor, Abl kinase inhibitor, VEGFR inhibitor, FGFR inhibitor, PDGFR inhibitor, Ephrin inhibitor and the like.

Examples of the tublin acting agent include, vinblastine, vindesine, vincristine, vinorelbine, paclitaxel (Taxol), docetaxel (Taxotere) and the like.

Examples of the DNA acting include chlorambucil, cyclophosphamide, melpharan, cisplatin, carboplatin, , dacarbazine (DTIC), oxaloplatin, bleomycin, doxorubicin (adriamycin), doxorubicin lipo (doxil), idarubicin, mitomycin, mitoxantrone, etoposide, camptothecin, CPT-11,10-hydroxy-7-ethyl-camptothecin (SN38), irinotecan, topotecan, 5-azacytidine, decitabine and the like.

Examples of the antimetabolite include 5-fluorouracil, fludarabine, hydroxyurea, cytarabine, methotrexate, capecitabine, gemcitabine (gemzar), tegafur-uracil mixture (UFT), clofarabine, nelarabine and the like.

Examples of the hormone therapeutic agents include leuprolide, goserelin, megestrol, tamoxifen, ICI182780, Tremifene, fadrozole, letrozole, flutamide, bicalutamide, testolactone, mitotane, prednisolone dexamethasone and the like.

Examples of the molecular targeted drugs include gefitinib (Iressa), erlotinib (Tarceva), lapatinib [(Tykerb), HKI-272, BIBW-2992, BMS-599626], imatinib [(Gleevec), STI571], dasatinib [(Sprycel), BMS-354825], nilotinib [(Tasigna), AMN107], sunitinib [(SUTENT), SU11248], sorafenib [(Nexabar), BAY43-9006], CHIR-258, vatalanib (PTK-787), R-1155777 (tipifarnib, zarnestra), rapamycin, temsirolimus, (CCI-779), bortezomib [(Velcade), PS-341], PR-171, NPI-0052, vorinostat [(Zolinza), suberanilohydroxamic acid, SAHA], valproic acid, MS-275, asparaginase, pegaspargase (Oncaspar) and the like.

Examples of the Flt-3 inhibitor include, CEP-701, PKC412, MLN518, CHIR-258, an indazole derivative represented by Formula (III)

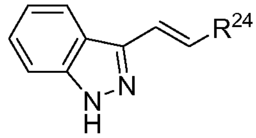

(III)

(wherein $R^{24}$ represents substituted or unsubstituted aryl or a substituted or unsubstituted heterocyclic group) or a pharmaceutically acceptable salt thereof, an indazole derivative represented by Formula (IIIa)

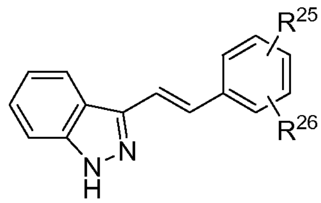

(IIIa)

[wherein $R^{25}$ represents $CONR^{27a}R^{27b}$ (wherein $R^{27a}$ and $R^{27b}$, which may be the same or different, each represent a hydrogen atom, substituted or unsubstituted lower alkyl, substituted or unsubstituted aryl, substituted or unsubstituted aralkyl, or a substituted or unsubstituted heterocyclic group, or $R^{27a}$ and $R^{27b}$ are combined together with the adjacent nitrogen atom thereto to form a substituted or unsubstituted heterocyclic group) or $NR^{28a}R^{28b}$ (wherein $R^{28a}$ represents substituted or unsubstituted lower alkylsulfonyl or substituted or unsubstituted arylsulfonyl and $R^{28b}$ represents a hydrogen atom or substituted or unsubstituted lower alkyl), and

$R^{26}$ represents a hydrogen atom, halogen, cyano, nitro, hydroxy, carboxy, lower alkoxycarbonyl, substituted or unsubstituted lower alkyl, substituted or unsubstituted lower alkoxy, substituted or unsubstituted lower alkanoyl, $CONR^{21a}R^{21b}$ (wherein $R^{29a}$ and $R^{21b}$, which may be the same or different, each represent a hydrogen atom, substituted or unsubstituted lower alkyl, substituted or unsubstituted aryl, substituted or unsubstituted aralkyl or a substituted or unsubstituted heterocyclic group, or $R^{29a}$ and $R^{29b}$ are combined together with the adjacent nitrogen atom thereto to form a substituted or unsubstituted heterocyclic group) or $NR^{30a}R^{30b}$ (wherein $R^{30a}$ and $R^{30b}$, which may be the same or different, each represent a hydrogen atom, substituted or unsubstituted lower alkyl, substituted or unsubstituted lower alkanoyl, substituted or unsubstituted aroyl, substituted or unsubstituted heteroaroyl, substituted or unsubstituted aralkyl, substituted or unsubstituted lower alkylsulfonyl or substituted or unsubstituted arylsulfonyl)], or a pharmaceutically acceptable salt thereof, an indazole derivative represented by Formula (IIIb)

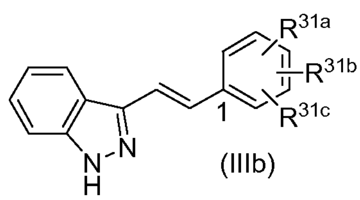

(IIIb)

{wherein $R^{31a}$, $R^{31b}$ and $R^{31c}$, which may be the same or different, each represent a hydrogen atom, halogen, nitro, nitroso, carboxy, cyano, substituted or unsubstituted lower alkyl, substituted or unsubstituted lower alkanoyl, substituted or unsubstituted lower alkoxycarbonyl, substituted or unsubstituted aryl, $NR^{32a}R^{32b}$ (wherein $R^{32a}$ and $R^{32b}$, which may be the same or different, each represent a hydrogen atom, substituted or unsubstituted lower alkyl, substituted or unsubstituted lower alkenyl, substituted or unsubstituted lower alkynyl, substituted or unsubstituted lower alkoxy, substituted or unsubstituted lower alkanoyl, substituted or unsubstituted aryl, substituted or unsubstituted aroyl, a substituted or unsubstituted heterocyclic group or substituted or unsubstituted heteroaroyl, or $R^{32a}$ and $R^{32b}$ are combined together with the adjacent nitrogen atom thereto to form a substituted or unsubstituted heterocyclic group) or $OR^{33}$ (wherein $R^{33}$ represents a hydrogen atom, substituted or unsubstituted lower alkyl, substituted or unsubstituted aryl, substituted or unsubstituted aroyl or a substituted or unsubstituted heterocyclic group)}, or a pharmaceutically acceptable salt thereof, an indazole derivative represented by Formula(IIIc)

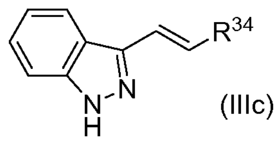

(IIIc)

{wherein $R^{34}$ represents a substituted or unsubstituted heterocyclic group [substituents in the substituted heterocyclic group may be the same or different, are 1 to 3 in number, and include oxo, formyl, carboxy, lower alkoxycarbonyl, substituted or unsubstituted lower alkyl, substituted or unsubstituted lower alkoxy, $CONR^{35a}R^{35b}$ (wherein $R^{35a}$ and $R^{35b}$, which may be the same or different, each represent a hydrogen atom or substituted or unsubstituted lower alkyl), $NR^{36a}R^{36b}$ (wherein $R^{36a}$ and $R^{36b}$, which may be the same or different, each represent a hydrogen atom, lower alkanoyl, lower alkoxycarbonyl, aralkyl, substituted or unsubstituted lower alkyl, substituted or unsubstituted aryl, substituted or unsubstituted aroyl or a substituted or unsubstituted heterocyclic group) or $-O(CR^{37a}R^{37b})_mO-$ (wherein $R^{37a}$ and $R^{37b}$, which may be the same or different, each represent a hydrogen atom or lower alkyl, m represents 2 or 3, and two terminal oxygen atoms are combined on the same carbon atom in the substituted heterocyclic group)]} or a pharmaceutically acceptable salt thereof, a pyrimidine derivative represented by Formula(IV)

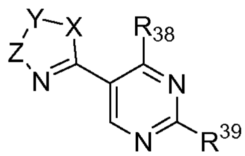

(IV)

<wherein -X-Y-Z represents $-O-CR^{40}=N-$ {wherein $R^{40}$ represents a hydrogen atom, hydroxy, carboxy, lower alkyl, lower alkyl substituted with 1 to 4 substituents which may be the same or different and selected from the substituent group C defined below [substituent group C: halogen, amino, aminosulfonyl, nitro, hydorxy, mercapto, cyano, formyl, carboxy, cabamoyl, lower alkanoyloxy, lower alkanoylamino, mono- or di-(lower alkyl)aminocarbonyl, lower alkoxycarbonyl, mono- or di-lower alkylamino, N-aryl-N-lower alkylamino, lower-alkylsulfonyl, lower alkylsulfynyl, mono- or di-(lower alkylsulfonyl)amino, mono- or di-(arylsulfonyl)amino, tri-lower alkylsilyl, lower alkylthio, aromatic heterocycle-alkylthio, lower alkanoyl, lower alkanoyl substituted with 1 to 3 substituents which may be the same or different and selected from the substituent group c defined below (substituent group c: halogen and hydroxy), lower alkoxy, lower alkoxy substituted with 1 to 3 substituents which may be the same or different and selected from the substituent group c defined above, aryloxy, aryloxy substituted with 1 to 3 substituents which may be the same or different and selected from the substituent group c defined above, aralkyloxy, or aralkyloxy substituted with 1 to 3 substituents which may be the same or different and selected from the substituent group c defined above; when the substituted lower alkyl is substituted methyl, substituted ethyl or substituted propyl, their substituents may be $-NR^{41a}R^{41b}$ (wherein $R^{41a}$ and $R^{41b}$, which may be the same or different, each represent a hydrogen atom, substituted or unsubstituted lower alkyl, substituted or unsubstituted lower cycloalkyl, substituted or unsubstituted lower alkenyl, substituted or unsubstituted lower alkynyl, substituted or unsubstituted aralkyl, substituted or unsubstituted aromatic heterocycle-alkyl, or substituted or unsubstituted aliphatic heterocycle-alkyl, substituted or unsubstituted aryl, substituted or unsubstituted aromatic heterocyclic group or substituted or unsubstituted aliphatic heterocyclic group)], lower cycloalkyl, lower cycloalkyl substituted with 1 to 4 substituents which may be the same or different and selected from the substituent group C defined above, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted aryl, a substituted or unsubstituted aromatic heterocyclic group, a substituted or unsubstituted aliphatic heterocyclic group, substituted or unsubstituted lower alkoxy, substituted or unsubstituted lower alkoxycarbonyl, substituted or unsubstituted lower alkylthio, substituted or unsubstituted lower alkanoyl, or $-C(=O)NR^{42a}R^{42b}$ (wherein $R^{42a}$ and $R^{42b}$, which may be the same or different, each represent a hydrogen atom, substituted or unsubstituted lower alkyl, substituted or unsubstituted lower cycloalkyl, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted aryl, a substituted or unsubstituted aromatic heterocyclic group or a substituted or unsubstituted aliphatic heterocyclic group, or $R^{42a}$ and $R^{42b}$ are combined together with the adjacent

nitrogen atom thereto to form a substituted or unsubstituted aliphatic heterocyclic group)}, -N=CR$^{40a}$-O- (wherein R$^{40a}$ has the same meaning as R$^{40}$ defined above), -O-N=CR$^{40b}$ (wherein $^{40b}$ has the same meaning as R$^{40}$ defined above), -O-C(=O)-NR$^{43}$- (wherein R$^{43}$ represents a hydrogen atom, lower alkyl, lower alkyl substituted with 1 to 4 substituents which may be the same or different and selected from the substituent group C defined above, lower cycloalkyl, lower cycloalkyl substituted with 1 to 4 substituents which may be the same or different and selected from the substituent group C defined above, or substituted or unsubstituted aliphatic heterocycle-alkyl), -N=N-NR$^{44}$- (wherein R$^{44}$ represents substituted or unsubstituted lower alkyl, substituted or unsubstituted lower cycloalkyl, or substituted or unsubstituted aliphatic heterocycle-alkyl), or -NR$^{44a}$-N=N- (wherein R$^{44a}$ has the same meaning as R$^{44}$ defined above),

R$^{38}$ represents -NR$^{45a}$R$^{45b}$ (wherein R$^{45a}$ and R$^{45b}$, which may be the same or different, each represent a hydrogen atom, substituted or unsubstituted lower alkyl, substituted or unsubstituted lower cycloalkyl, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted monocyclic aryl, substituted or unsubstituted monocyclic aromatic heterocyclic group or substituted or unsubstituted aliphatic heterocyclic group or R$^{45a}$ and R$^{45b}$ form a substituted or unsubstituted aliphatic heterocyclic group together with the adjacent nitrogen atom thereto, and when one of R$^{45a}$ or R$^{45b}$ is a hydrogen atom, the other of R$^{45a}$ or R$^{45b}$ is not substituted or unsubstituted pyrazol-3-yl or substituted or unsubstituted 1,2,4-triazol-3-yl) or -OR$^{46}$ (wherein R$^{46}$ represents a hydrogen atom, substituted or unsubstituted lower alkyl, substituted or unsubstituted lower cycloalkyl, substituted or unsubstituted lower alkenyl, substituted or unsubstituted lower alkynyl, substituted or unsubstituted aralkyl, substituted or unsubstituted aromatic heterocycle-alkyl, substituted or unsubstituted aliphatic heterocycle-alkyl, substituted or unsubstituted aryl, substituted or unsubstituted aromatic heterocyclic group or substituted or unsubstituted aliphatic heterocyclic group), and R$^{39}$ represents -NR$^{47a}$R$^{47b}$ {wherein R$^{47a}$ and R$^{47b}$, which may be the same or different, each represent a hydrogen atom, lower alkyl, lower alkyl substituted with 1 to 4 substituents which may be the same or different and selected from the substituent group D defined below [substituent group D: halogen, amino, aminosulfonyl, nitro, hydroxy, mercapto, cyano, formyl, carboxy, cabamoyl, lower alkanoyloxy, lower alkanoylamino, mono- or di-(lower alkyl)aminocarbonyl, lower alkoxycarbonyl, mono- or di-lower alkylamino, N-aryl-N-lower alkylamino, lower-alkylsulfonyl, lower alkylsulfynyl, mono- or di-(lower alkylsulfonyl)amino, mono- or di-(arylsulfonyl)amino, tri-lower alkylsilyl, lower alkylthio, aromatic heterocycle-alkylthio, lower alkanoyl, lower alkanoyl substituted with 1 to 3 substituents which may be the same or different and selected from the substituent group c defined below (substituent group c: halogen or hydroxy), lower alkoxy, lower alkoxy substituted with 1 to 3 substituents which may be the same or different and selected from the substituent group c defined above, aryloxy, aryloxy substituted with 1 to 3 substituents which may be the same or different and selected from the substituent group c defined above, aralkyloxy, or aralkyloxy substituted with 1 to 3 substituents which may be the same or different and selected from the substituent group c defined above], lower cycloalkyl, lower cycloalkyl substituted with 1 to 4 substituents which may be the same or different and selected from the substituent group D as defined above, substituted or unsubstituted lower alkenyl, substituted or unsubstituted lower alkynyl, substituted or unsubstituted aliphatic heterocycle-alkyl, substituted or unsubstituted monocyclic aryl, or substituted or unsubstituted aliphatic heterocyclic group or R$^{47a}$ and R$^{47b}$ are combined together with the adjacent nitrogen atom thereto to form a substituted or unsubstituted aliphatic heterocyclic group or substituted or unsubstituted aromatic heterocyclic group; but R$^{47a}$ and R$^{47b}$ do not simultaneously be hydrogen atoms}, -NR$^{48}$CR$^{49a}$R$^{49b}$-Ar {wherein R$^{48}$ represents a hydrogen atom, lower alkyl or lower cycloalkyl, R$^{49a}$ and R$^{49b}$, which may be the same or different, each represent a hydrogen atom, lower alkyl, lower alkyl substituted with 1 to 3 substituents which may be the same or different and selected from the substituent group d defined below (substituent group d: halogen, hydorxy and hydroxymethyl), lower cycloalkyl, or lower cycloalkyl substituted with 1 to 3 substituents which may be the same or different and selected from the substituent group d defined above, Ar represents aryl, aryl substituted with 1 to 3 substituents which may be the same or different and selected from the substituent group E defined below [substituent group E: halogen, amino, nitro, hydorxy, mercapto, cyano, carboxy, aminosulfonyl, lower alkyl, lower alkyl substituted with 1 to 3 substituents which may be the same or different and selected from the substituent group d defined above, lower cycloalkyl, lower cycloalkyl substituted with 1 to 3 substituents which may be the same or different and selected from the substituent group d defined above, lower alkoxy, lower alkylthio, mono- or di-lower alkylamino, lower alkanoylamino, mono- or di-(lower alkylsulfonyl)amino, lower alkoxycarbonylamino, aliphatic heterocycle-alkyloxy and alkylenedioxy], aromatic heterocyclic group or aromatic heterocyclic group substituted with 1 to 3 substituents which may be the same or different and selected from the substituent group E defined above} or -NR$^{48}$CR$^{49a}$R$^{49b}$CR$^{50a}$R$^{50b}$-Ar (wherein R$^{48}$, R$^{49a,}$ R$^{49b}$ and Ar have the same meanings as defined above, respectively, and R$^{50a}$ and R$^{50b}$ have the same meaning as R$^{49a}$ and R$^{49b}$ defined above, respectively)>, or a pharmaceutically acceptable salt thereof,

isoindolinone phthalimide derivative represented by formula (V)

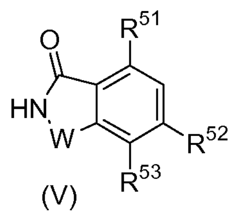

(V)

[wherein W represents -C(=O)- or -CHR$^{54}$-(wherein $R^{54}$ represents a hydrogen atom, hydroxy, substituted or unsubstituted lower alkyl or substituted or unsubstituted lower alkoxy),
$R^{51}$ represents

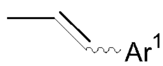

{wherein, $Ar^1$ reprsents aryl, aryl substituted with 1 or 2 substituents which may be the same or different and selected from the substituent group F described below, monocyclic aromatic heterocyclic group or monocyclic aromatic heterocyclic group substituted with 1 or 2 substituents which may be the same or different selected from the substituent group F described below; substituent group F [halogen, nitro, hydroxy, cyano, carboxy, lower alkoxycarbonyl, substituted or unsubstituted lower alkyl, substituted or unsubstituted lower alkoxy, substituted or unsubstituted lower alkanoyl, -CONR$^{55a}$R$^{55b}$ (wherein, $R^{55a}$ and $R^{55b}$, which may be the same or different, each represent a hydrogen atom, substituted or unsubstituted lower alkyl, substituted or unsubstituted aryl or substituted or unsubstituted aralkyl, or $R^{55a}$ and $R^{55b}$ are combined together with the adjacent nitrogen atom thereto to form a substituted or unsubstituted heterocyclic group) or -NR$^{56a}$R$^{56b}$ (wherein, $R^{56a}$ and $R^{56b}$, which may be the same or different, each represent a hydrogen atom, substituted or unsubstituted lower alkyl, substituted or unsubstituted lower alkanoyl, substituted or unsubstituted lower alkylsulfonyl, substituted or unsubstituted aryl, substituted or unsubstituted aralkyl, substituted or unsubstituted aroyl, substituted or unsubstituted arylsulfonyl or substituted or unsubstituted heteroaroyl)]},
$R^{52}$ represents a hydrogen atom or

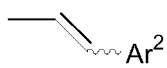

(wherein $Ar^2$ has the same meaning as $Ar^1$ defined above), $R^{53}$ represents a hydrogen atom, halogen, substituted or unsubstituted lower alkyl, substituted or unsubstituted lower alkanoyl, substituted or unsubstituted aryl, -NR$^{57a}$R$^{57b}$ [wherein $R^{57a}$ and $R^{57b}$, which may be the same or different, each represent a hydrogen atom, substituted or unsubstituted lower alkyl or -C(=O)-R$^{58}$ (wherein $R^{58}$ represents a hydrogen atom, substituted or unsubstituted lower alkyl, substituted or unsubstituted lower alkoxy or substituted or unsubstituted aralkyl)] or

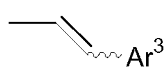

(wherein $Ar^3$ has the same meaning as $Ar^1$ defined above) provided that when $R^{52}$ is a hydrogen atom, and $Ar^1$ is aryl, aryl substituted with 2 lower alkoxy, or aryl substituted with only one lower alkyl or lower alkoxy, $R^{53}$ is not a hydrogen atom] or a pharmaceutically acceptable salt thereof.

In the definition for each groups in Formulae (III), (IIIa), (IIIb), (IIIc), (IV) and (V),

(i) Examples of halogen include each atoms of fluorine, chlorine, bromine and iodine;
(ii) Examples of lower alkyl and the lower alkyl moieties of lower alkoxy, lower alkoxycarbonyl, lower alkoxycarbonylamino, lower alkoxycarbonyl substituted lower alkyl and lower alkylsulfonyl include, for example, linear, branched or cyclic alkyl or alkyl comprising these alkyls in combination, having 1 to 10 carbon atoms More specific examples

thereof are as follows.

(ii-a) Examples of the linear or branched lower alkyl include, for example, methyl, ethyl, *n*-propyl, isopropyl, *n*-butyl, isobutyl, sec-butyl, *tert*-butyl, *n*-pentyl, neopentyl, *n*-hexyl, *n*-heptyl, n-octyl, *n*-nonyl, and *n*-decyl;
(ii-b) Examples of the cyclic lower alkyl include, for example, cyclopropyl, cyclobutyl, cyclopentyl, cyclohexyl, cycloheptyl, cyclooctyl, cyclodecyl, noradamantyl, adamantyl, bicyclo[2.2.1]heptyl, bicyclo[2.2.2]octyl, bicyclo [3.3.0]octyl, and bicyclo[3.3.1]nonyl;
(ii-c) Examples of the lower alkyl comprising linear or branched alkyl and cyclic alkyl include, for example, cyclopropylmethyl, cyclopentylmethyl, and cyclooctylethyl.

(iii) The lower alkoxycarbonyl substituted lower alkyl and the alkylene moiety of the aralkyl has the same meaning as the group formed by removing one hydrogen atom from the linear or branched lower alkyl (ii-a) in the definition of the lower alkyl defined above.
(iv) Examples of the lower alkenyl include, for example, linear or branched alkenyl having 2 to 10 carbon atoms such as vinyl, allyl, 1-propenyl, 1-butenyl, 3-butenyl, 2-pentenyl, 4-pentenyl, 2-hexenyl, 5-hexenyl, 2-decenyl, and 9-decenyl.
(v) Examples of the lower alkynyl include, for example, linear or branched alkynyl having 2 to 10 carbon atoms such as ethynyl, 2-propynyl, 3-butynyl, 4-pentynyl, 5-hexynyl, and 9-decynyl.
(vi) Examples of the aryl, aryl moiety of the aralkyl, aroyl, aroylamino, and arylsulfonyl include , for example, monocyclic aryl or condensed aryl in which 2 or more rings are condensed; more specific examples thereof include aryl having 6 to 14 carbon atoms as ring-constituting members, such as phenyl, naphthyl indenyl, and anthryl.
(vii) Examples of the lower alkanoyl include, for example, linear, branched, cyclic or a combination of these alkanoyl having 1 to 8 carbon atoms, such as formyl, acetyl, propionyl, butyryl, isobutyryl, valeryl, isovaleryl, pivaloyl, hexanoyl, heptanoyl, octanoyl, cyclopropylcarbonyl, cyclobutylcarbonyl, cyclopentylcarbonyl, cyclopropylmethylcarbonyl, cyclohexylcarbonyl, 1-methylcyclopropylcarbonyl, and cycloheptylcarbonyl;
(viii) examples of the heterocyclic group include, for example, aromatic heterocyclic group, and aliphatic heterocyclic group;

(viii-a) Examples of the aromatic heterocyclic group include, for example, monocyclic aromatic heterocyclic group or condensed aromatic heterocyclic group in which 2 or more rings are condensed. The type and number of the heteroatom contained in aromatic heterocyclic group are not specifically limited and the aromatic heterocyclic group may contain, for example, one or more heteroatoms selected from the group consisting of nitrogen atom, sulfur atom and oxygen atom. More specific examples include aromatic heterocyclic group having 5 to 14 carbon atoms as ring-constituting members, such as furyl, thienyl, pyrrolyl, imidazolyl, pyrazolyl, triazolyl, tetrazolyl, oxazolyl, oxadiazolyl, thiazolyl, pyridyl, pyrazinyl, pyrimidinyl, pyridazinyl, triazinyl, indolyl, indazolyl, benzimidazolyl, benzoxazolyl, benzothiazolyl, quinolyl, isoquinolyl, phthalazinyl, naphthyridinyl, quinoxalinyl, quinazolinyl, cinnolinyl, purinyl and coumarinyl.
(viii-b) Examples of the aliphatic heterocyclic group include, for example, monocyclic aliphatic heterocyclic group or condensed aliphatic heterocyclic group in which two or more rings are condensed. The type and number of the heteroatom contained in aliphatic heterocyclic groups are not specifically limited and the aliphatic heterocyclic group may contain, for example, one or more heteroatoms selected from the group consisting of nitrogen atom, sulfur atom and oxygen atom. More specific examples include, for example, pyrrolidinyl, 2,5-dioxopyrrolidinyl, thiazolidinyl, oxazolidinyl, piperidyl, 1,2-dihydropyridyl, piperazinyl, homopiperazinyl, morpholinyl, thiomorpholinyl, pyrazolinyl, oxazolinyl, dioxolanyl, tetrahydropyranyl, tetrahydrothiopyranyl, tetrahydrofuryl, tetrahydroquinolyl, tetrahydroisoquinolyl, tetrahydroquinoxalinyl, octahydroquinolyl, dihydroindolnyl and 1,3-dioxoisoindolinyl.

(vii) Examples of the heterocyclic group formed together with the adjacent nitrogen atom include, for example, 5- or 6-membered monocyclic aliphatic heterocyclic group containing at least one nitrogen atom (the monocyclic aliphatic heterocyclic group may further contain any other of a nitrogen atom, an oxygen atom and a sulfur atom) and bicyclic or tricyclic condensed heterocyclic group containing at least one nitrogen atom in which 3- to 8-membered rings are condensed (the condensed heterocyclic group may further contain any other of a nitrogen atom, an oxygen atom and a sulfur atom). More specific examples include, for example, pyrrolidinyl, piperidino, piperazinyl, morpholino, thiomorpholino, homopiperidino, homopiperazinyl, tetrahydropyridyl, tetrahydroquinolyl, and tetrahydroisoquinolyl.
(x) The heteroaryl moiety of the heteroaroyl has the same meaning as the aromatic heterocyclic group (viii-a) defined above.
(ix) Examples of the substituents in the substituted lower alkyl, the substituted lower alkoxy, the substituted lower

alkenyl, the substituted lower alkynyl, the substituted lower alkanoyl, the substituted lower alkoxycarbonyl, and the substituted lower alkylsulfonyl, which may be the same or different and in number of 1 to 3, include

(xi-a) hydroxy,
(xi-b) lower alkoxy,
(xi-c) oxo,
(xi-d) carboxy,
(xi-e) lower alkoxycarbonyl,
(xi-f) heteroaroyl,
(xi-g) arylsulfonyl,
(xi-h) substituted or unsubstituted aryl [the substituent(s) in the substituted aryl is for example, carboxy, lower alkoxy and lower alkoxycarbonyl],
(xi-i) a substituted or unsubstituted heterocyclic group [the substituent(s) in the substituted heterocyclic group is for example, carboxy, lower alkoxy and lower alkoxycarobnyl],
(xi-j) $CONR^{59a}R^{59b}$ (wherein $R^{59a}$ and $R^{59b}$, which may be the same or different, each represent a hydrogen atom, substituted or unsubstituted lower alkyl [the substituent(s) in the substituted lower alkyl, which is 1 to 3 in number, is for example, halogen, hydroxy oxo, nitro, cyano, carboxy, lower alkanoyl, lower alkoxycarbonyl, aroyl, substituted or unsubstituted lower alkoxy (the substituent(s) in the substituted lower alkoxy, which is 1 to 3 in number, is for example, hydroxy)], or $R^{59a}$ and $R^{59b}$ are combined together with the adjacent nitrogen atom thereto to form a substituted or unsubstituted heterocyclic group [the substituent(s) in the substituted heterocyclic group formed together with the adjacent nitrogen atom, which is 1 to 3 in number, is for example, halogen, hydroxy, oxo, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example, hydroxy), substituted or unsubstituted lower alkoxy (the substituent(s) in the substituted lower alkoxy, which is 1 to 3 in number, is for example, hydroxy)]},
(xi-k) $NR^{60a}R^{60b}$ (wherein $R^{60a}$ and $R^{60b}$ have the same meanings as $R^{59a}$ and $R^{59b}$ defined above, respectively),
(xi-1) lower alkanoylamino,
(xi-m) N-lower alkanoyl-N-lower alkylamino, and the like.

In the definition of the substituents (xi) in the substituted lower alkyl, the substituted lower alkoxy, the substituted lower alkenyl, the substituted lower alkynyl, the substituted lower alkanoyl, the substituted lower alkoxycarbonyl and the substituted lower alkylsulfonyl, the halogen has the same meaning as (i) defined above, the lower alkyl and the lower alkyl moieties of the lower alkoxy, the lower alkoxycarbonyl, and the N-lower alkanoyl-N-lower alkylamino have the same meanings as (ii) defined above, the alkylene moiety of the aralkyl has the same meaning as (iii) defined above, the aryl, and the aryl moieties of the aralkyl, aroyl and the arylsulfonyl have the same meaning as (vi) defined above, the lower alkanoyl and the lower alkanoyl moieties of the lower alkanoylamino and the N-lower alkanoly-N-lower alkylamino have the same meanings as (vii) defined above, the heterocyclic group has the same meaning as (viii) defined above, the heterocyclic group formed together with the adjacent nitrogen atom has the same meaning as (x) defined above.
(xii) Examples of the substituents in the substituted aryl, the substituted aroyl, the substituted aralkyl, the substituted arylsulfonyl, the substituted heteroaroyl, the substituted heterocyclic group and the substituted heterocyclic group formed together with the adjacent nitrogen atom, which may be the same or different and is 1 to 3 in number, include

(xii-a) halogen,
(xii-b) nitro,
(xii-c) nitroso
(xii-d) carboxy,
(xii-e) substituted or unsubstituted lower alkyl [the substituent(s) in the substituted lower alkyl has the same meaning as (xi) defined above],
(xii-f) substituted or unsubstituted lower alkenyl [the substituent(s) in the substituted lower alkenyl has the same meaning as (xi) defined above],
(xii-g) substituted or unsubstituted lower alkynyl [the substituent(s) in the substituted lower alkynyl has the same meaning as (xi) defined above],
(xii-h) substituted or unsubstituted lower alkoxycarbonyl [the substituent(s) in the substituted lower alkoxycarbonyl has the same meaning as (xi) defined above],
(xii-i) substituted or unsubstituted lower alkanoyl [the substituent(s) in the substituted lower alkanoyl has the same meaning as (xi) defined above],

(xii-j) substituted or unsubstituted aryl [the substituent(s) in the substituted aryl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy) and substituted or unsubstituted lower alkoxy (the substituent(s) in the substituted lower alkoxy, which is 1 to 3 in number, is for example hydroxy)],

(xii-k) $NR^{61a}R^{61b}$ {wherein $R^{61a}$ and $R^{61b}$, which may be the same or different, each represent a hydrogen atom, lower alkylsulfonyl, substituted or unsubstituted lower alkyl [the substituent(s) in the substituted lower alkyl has the same meaning as (xi) defined above], substituted or unsubstituted lower alkenyl [the substituent (s) in the substituted lower alkenyl has the same meaning as (xi) defined above], substituted or unsubstituted lower alkynyl [the substituent(s) in the substituted lower alkynyl has the same meaning as (xi) defined above], substituted or unsubstituted lower alkoxy [the substituent(s) in the substituted lower alkoxy has the same meaning as (xi) defined above], substituted or unsubstituted lower alkanoyl [the substituent(s) in the substituted lower alkanoyl has the same meaning as (xi) defined above], substituted or unsubstituted aryl [the substituent(s) in the substituted aryl which is 1 to 3 in number, is for example, halogen, hydroxy, nitro, cyano, carboxy, lower alkanoyl, lower alkoxycarbonyl, aralkyl, aroyl, substituted or unsubstituted lower alkyl (wherein the substituent (s) in the substituted lower alkyl, which is 1 to 3 in number, is for example hydroxy), substituted or unsubstituted lower alkoxy (the substituent(s) in the substituted lower alkoxy, which is 1 to 3 in number, is for example hydroxy)], substituted or unsubstituted aroyl [the substituent(s) in the substituted aroyl which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy) or substituted or unsubstituted lower alkoxy (the substituent(s) in the substituted lower alkoxy, which is 1 to 3 in number, is for example hydroxy)] or substituted or unsubstituted heterocyclic group [the substituent(s) in the substituted heterocyclic group, which is 1 to 3 in number, is for example halogen, hydroxy, nitro, cyano, carobxy, lower alkanoyl, lower alkoxycarbonyl, aralkyl, aroyl, substituted or unsubstituted lower alkyl (the substituent(s) in the substituted lower alkyl, which is 1 to 3 in number, is for example hydroxy), substituted or unsubstituted lower alkoxy (the substituent(s) in the substituted lower alkoxy, which is 1 to 3 in number, is for example hydroxy)] or $R^{61a}$ and $R^{61b}$ are combined together with the adjacent nitrogen atom thereto to form a substituted or unsubstituted heterocyclic group [the substituent(s) in the substituted heterocyclic group formed together with the adjacent nitrogen atom, which is 1 to 3 in number, is for example, halogen, amino, nitro, hydroxy, oxo, cyano, carboxy, lower alkoxycarbonyl, aralkyl, aroyl, heteroaroyl, substituted or unsubstituted lower alkyl (the substituent(s) in the substituted lower alkyl, which is 1 to 3 in number, is for example, hydroxy, lower alkoxy and the like), substituted or unsubstituted lower alkoxy (the substituent(s) in the substituted lower alkoxy, which is 1 to 3 in number, is for example, hydroxy, lower alkoxy and the like), substituted or unsubstituted lower alkanoyl (the substituent(s) in the substituted lower alkanoyl, which is 1 to 3 in number, is for example, amino, hydroxy, lower alkoxy, lower alkanolyamino, N-lower alkanoyl-N-lower alkylamino and the like), substituted or unsubstituted aliphatic heterocycle-carbonyl (the substituent(s) in the substituted aliphatic heterocycle-carbonyl, which is 1 to 3 in number, is for example, halogen, hydroxy, oxo, lower alkyl, lower alkoxy and the like)]},

(xii-1) $CONR^{62a}R^{62b}$ (wherein $R^{62a}$ and $R^{62b}$ have the same meanings as $R^{61a}$ and $R^{61b}$ defined above, respectively),

(xii-m) $OR^{63}$ {wherein $R^{63}$ represents a hydrogen atom, substituted or unsubstituted lower alkyl, [the substituent (s) in the substituted lower alkyl has the same meaning as (xi) defined above], substituted or unsubstituted aryl [the substituent(s) in the substituted aryl, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example, hydroxy), substituted or unsubstituted lower alkoxy (the substituent(s) in the substituted lower alkoxy, which is 1 to 3 in number, is for example, hydroxy)], or a substituted or unsubstituted heterocyclic group (the substituent(s) in the substituted heterocyclic group, which is 1 to 3 in number, is for example, halogen, hydroxy, nitro, cyano, carboxy, lower alkanoyl, lower alkoxycarbonyl, aralkyl, aroyl, substituted or unsubstituted lower alkyl (the substituent(s) in the substituted lower alkyl, which is 1 to 3 in number, is for example, hydroxy), substituted or unsubstituted lower alkoxy (the substituent(s) in the substituted lower alkoxy, which is 1 to 3 in number, is for example, hydroxy))},

(xii-n) heteroaroyl,

(xii-o) substituted or unsubstituted aliphatic heterocycle-carbonyl (the substituent(s) in the substituted aliphatic heterocycle-carbonyl, which is 1 to 3 in number, is for example, halogen, hydroxy, oxo, lower alkyl, and lower alkoxy).

The substituent(s) in the substituted aliphatic heterocyclic group, and the substituted heterocyclic group formed together with the adjacent nitrogen atom may be, in addition to (xii-a) to (xii-o), the following (xii-p) or (xii-q):

(xii-p) oxo

(xii-q) -$O(CR^{64a}R^{64b})_pO$- (wherein $R^{64a}$ and $R^{64b}$, which may be the same or different, each represent a hydrogen atom, or lower alkyl, and p represents 2 or 3, and the two terminal oxygen atoms are combined on the same carbon atom in the substituted heterocyclic group and the substituted heterocyclic group formed together with the adjacent nitrogen atom).

In the definition of the substituents (xii) in the substituted aryl, the substituted aroyl, the substituted aralkyl, the substituted arylsulfonyl, the substituted heteroaroyl, the substituted heterocyclic group and the substituted heterocyclic group formed together with the adjacent nitrogen atom, the halogen has the same meaning as (i) defined above; the lower alkyl and the lower alkyl moieties of the N-lower alkanoyl-N-lower alkyl, the lower alkoxy, the lower alkoxycarbonyl, and the lower alkylsulfonyl have the same meanings as (ii) defined above, the alkylene moiety of the aralkyl has the same meaning as (iii) defined above, the lower alkenyl has the same meaning as (iv) defined above, the lower alkynyl has the same meaning as (v) defined above, the aryl and the aryl moieties of the aroyl and the aralkyl have the same meanings as (vi) defined above, respectively, the lower alkanoyl and the lower alkanoyl moiety of the lower alkanyolamino and the N-lower alkanoyl-N-lower alkylamino have the same meanings as (vii) defined above, the heterocyclic group has the same meaning as (viii) defined above, the aliphatic heterocyclic moiety of the aliphatic heterocycle-carbonyl has the same meaning as (viii-b) defined above, the heterocyclic group formed together with the adjacent nitrogen atom has the same meaning as (ix) defined above, and the heteroaroyl has the same meaning as (x) defined above.

Indazole derivatives represented by Formulae (III), (IIIa), (IIIb) and (IIIc) or a pharmaceutically acceptable salt thereof can be synthesized according to the method described in, for example, WO2005/012257 or WO2005/012258.

Pyrimidine derivatives represented by Formula (IV) or a pharmaceutically acceptable salt thereof can be synthesized according to the method described in, for example, W02005/095382.

Isoindolinone phthalimide derivatives represented by Formula (V) or a pharmaceutically acceptable salt thereof can be synthesized according to the method described in, for example, WO2005/095341.

Examples of the differentiation-inducing agent include, for example, all-trans retinoic acid, arsenic trioxide, thalidomide, lenalidomide, bexarotene (targretin) and the like.

Examples of the osteoclastic inhibitor include, for example, bisphosphonate (zoledronic acid, Zometa) and the like.

There are concerns that when administered solely, the above compounds may not give sufficient treatment results. Also, high-dose administration of the above compounds may cause side effects. By combining the above compounds together with the Hsp90 family protein inhibitors, the present invention provides better treatment results than administering compounds alone. Further, because it is possible to obtain better treatment results by combining Hsp90 family protein inhibitor with the above compounds when compared to single administration, at least either of Hsp90 family protein inhibitors or the above compounds can be used in a low dosage. Therefore, the present invention not only provides sufficient effect of treatment but also decreases side effects.

Compounds (I) and (IA) or a pharmaceutically acceptable salt thereof used in the present invention can be synthesized based on the method described in, for example, W02005/000778.

Compound (II) or a pharmaceutically acceptable salt thereof used in the present invention can be synthesized based on the method described in, for example, W02005/063222.

Specific examples of the compounds used in the present invention will be described in the following Table 1 and 2 but the present invention is not limited to them. In the follwing tables, Ph represents phenyl.

Compounds 1 to 22 described in Table 1 can be synthesized by a method described in W02005/000778. Compounds 23 to 37 described in Table 2 can be synthesized by a method described in W02005/063222.

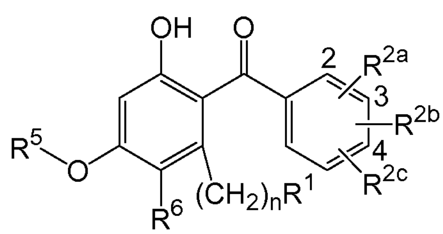 (I-i)

Table 1

| Compound | $R^1$ | n | $R^{2a}$ | $R^{2b}$ | $R^{2c}$ | $R^5$ | $R^6$ |
|---|---|---|---|---|---|---|---|
| 1 | $OCH_3$ | 2 | H | H | H | H | H |
| 2 | $OCH_3$ | 2 | H | H | H | H | Br |
| 3 | $OCH_3$ | 2 | H | H | H | H | $COCH_3$ |
| 4 | $CO_2CH_3$ | 1 | 3-$OCH_3$ | H | H | H | $CH_2CH_3$ |
| 5 | $OCH_3$ | 2 | 4-$OCH_3$ | H | H | H | $CH_2CH_3$ |
| 6 | $OCH_3$ | 2 | 4-$NO_2$ | H | H | H | $CH_2CH_3$ |
| 7 | $OCH_2CH_2OCH_3$ | 2 | 4-$OCH_3$ | H | H | H | $CH_2CH_3$ |
| 8 | $CON(CH_3)CH_2CH_2OH$ | 1 | 4-$OCH_3$ | H | H | H | $CH_2CH_3$ |
| 9 | 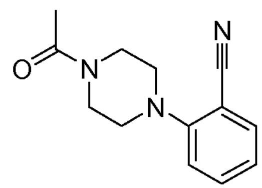 | 1 | 4-$OCH_3$ | H | H | H | $CH_2CH_3$ |
| 10 | $CO_2CH_3$ | 1 | 4-$OCH_3$ | H | H | $CH_2CH{=}CH_2$ | H |
| 11 | 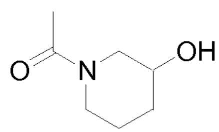 | 1 | 4-$OCH_3$ | H | H | H | $CH_2CH_3$ |
| 12 | 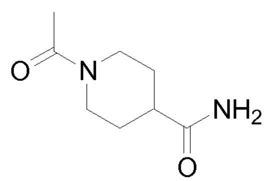 | 1 | 4-$OCH_3$ | H | H | H | $CH_2CH_3$ |

(continued)

| Compound | $R^1$ | n | $R^{2a}$ | $R^{2b}$ | $R^{2c}$ | $R^5$ | $R^6$ |
|---|---|---|---|---|---|---|---|
| 13 | 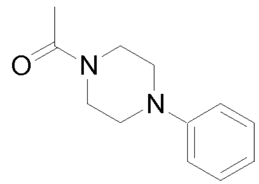 | 1 | 4-$OCH_3$ | H | H | H | $CH_2CH_3$ |
| 14 | 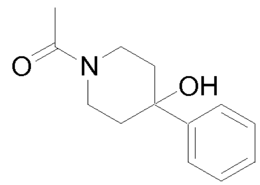 | 1 | 4-$OCH_3$ | H | H | H | $CH_2CH_3$ |
| 15 | $OCH_2CH(OH)CH_2OH$ | 2 | 2-F | 4-$OCH_3$ | H | H | $CH_2CH_3$ |
| 16 | 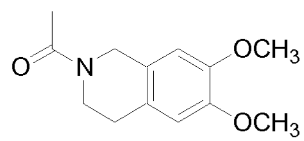 | 1 | 4-$OCH_3$ | H | H | H | $CH_2CH_3$ |
| 17 | $OCH_2CH(OH)CH_2OH$ | 2 | 3 - 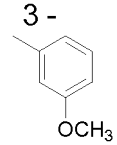 | 4-$OCH_3$ | H | H | $CH_2CH_3$ |
| 18 | $CON(CH_2CH_2OH)_2$ | 1 | 4-$OCHF_2$ | H | H | H | $CH_2CH_3$ |
| 19 | $CON(CH_2CH_2OH)CH_2CH_2OCH_3$ | 1 | 4-$SCH_3$ | H | H | H | $CH_2CH_3$ |
| 20 | $CON(CH_2CH_2OH)_2$ | 1 | 4-$SO_2CH_3$ | H | H | H | $CH_2CH_3$ |
| 21 | $CON(CH_2CH_2OH)CH_2CH_2OCH_3$ | 1 | 3- 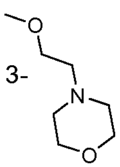 | 4-$OCH_3$ | H | H | $CH_2CH_3$ |

(continued)

| Compound | $R^1$ | n | $R^{2a}$ | $R^{2b}$ | $R^{2c}$ | $R^5$ | $R^6$ |
|---|---|---|---|---|---|---|---|
| 22 | $CON(CH_2CH_2OCH_3)_2$ | 1 | 3-$OCH_3$ | 4- 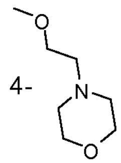 | H | H | $CH_2CH_3$ |

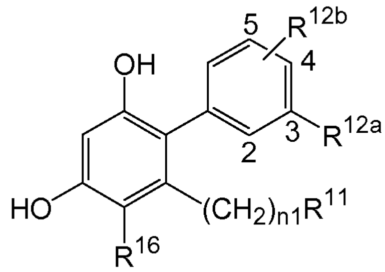

(II-i)

Table 2

| Compound | $R^{11}$ | n1 | $R^{12a}$ | $R^{12b}$ | $R^{16}$ |
|---|---|---|---|---|---|
| 23 | $CO_2CH_3$ | 1 | H | H | H |
| 24 | $CO_2CH_3$ | 1 | $CH=CHCOCH_3$ | H | H |
| 25 | $CO_2CH_3$ | 1 | $(CH_2)_2COCH_3$ | H | H |
| 26 | $CO_2CH_3$ | 1 | $COCH_3$ | H | H |
| 27 | $CONH(CH_2)_2N(CH_3)_2$ | 1 | H | H | Br |
| 28 | 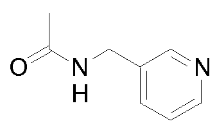 | 1 | H | H | Br |
| 29 | 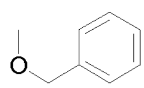 | 1 | H | H | Br |
| 30 | $CONH_2$ | 0 | H | H | Br |
| 31 | $CH=CHCO_2CH_3$ | 0 | H | H | Br |
| 32 | OH | 0 | H | H | Br |
| 33 | $CO_2CH_3$ | 1 | $CH_2CH_2CO_2H$ | H | Br |
| 34 | $CO_2CH_3$ | 1 | H | H | $CH_2Ph$ |
| 35 | $CO_2CH_3$ | 1 | H | 4-OPh | H |
| 36 | $OCH_2CONHCH_2CH_2OH$ | 3 | H | H | $CH_2CH_3$ |
| 37 | $OCH_2CH(OH)CH_2OH$ | 2 | 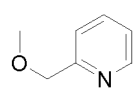 | H | $CH_2CH_3$ |

The pharmaceutical composition of the present invention can be used in treatment of any cancer, such as cancer derived from hematopoietic tumor (for example, acute myeloid leukemia, chronic myeloid leukemia, acute lymphocytic leukemia, chronic lymphocytic leukemia, multiple myeloma, or lymphoma), breast cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma, or cancer derived from brain tumor. Among them, the pharmaceutical composition is preferably used for acute myeloid leukemia, multiple myeloma, lung cancer, breast cancer, chronic myeloid leukemia, renal cancer, gastric cancer, and prostatic cancer, or the like.

The effect of the pharmaceutical composition of the present invention can be examined by analyzing the result of in vitro cell growth-inhibiting activity assay by using the isobologram method [International Journal of Radiation Oncology, Biology, Physics, Vol. 5, p. 85-91 (1979)].

The effect of the pharmaceutical composition of the present invention can also be examined by measuring in vivo antitumor activity using an animal model.

As the animal model, a model prepared by transplanting a cultured cell line derived from cancer tissue into immunodeficient mice such as nude mice can be used.

The effect of the pharmaceutical composition of the present invention can be evaluated by comparing the effect of single administration of an Hsp90 family protein inhibitor and of single administration of a combined compound with the effect of the pharmaceutical composition of the present invention using this animal model.

Examples of the cultured cells to be used include NCI-H596 cells, PC-9 cells, KPL-4 cells, BT-474 cells, NCI-H929 cells, MOLM-13 cells, K-562 cells and VMRC-RCZ cells. NCI-H596 and PC-9 are cells derived from patients with non-small-cell lung cancer and can provide a model of lung cancer. KPL-4 and BT-474 are cells derived from patients with breast cancer and can provide a model of breast cancer. NCI-H929 is cells derived from patients with multiple myeloma and can provide a model of multiple myeloma. MOLM-13 is cells derived from patients with acute myeloid leukemia and can provide a model of acute myeloid leukemia. K-562 is cells derived from patients with chronic myeloid leukemia and can provide a model of chronic myeloid leukemia. VMRC-RCZ is cells derived from patients with renal cancer and can provide a model of renal cancer.

The pharmaceutical composition of the present invention can be used, administered or produced as a single preparation (mixture) or as a combination of plural preparations, so long as it is prepared to contain an Hsp90 inhibitor and at least one compound to be combined with the Hsp90 inhibitor. These pharmaceutical compositions are preferably in a unit dosage form suitable for oral administration or parenteral administration such as injection. When they are used or administered as a combination of plural preparations, the combined preparations may be used or administered simultaneously or separately with an interval.

These preparations can be prepared according to conventional methods using, in addition to the active ingredients, pharmaceutically acceptable diluents, excipients, disintegrators, lubricants, binders, surfactants, water, physiological saline, vegetable oils, solubilizing agents, isotonic agents, preservatives, antioxidants, etc.

Tablets can be prepared using excipients such as lactose, disintegrators such as starch, lubricants such as magnesium stearate, binders such as hydroxypropyl cellulose, surfactants such as fatty acid ester, plasticizers such as glycerin, etc. in a conventional manner.

Injections can be prepared using water, physiological saline, vegetable oils, solvents, solubilizing agents, isotonic agents, preservatives, antioxidants, etc. in a conventional manner.

Compounds (I), (IA) and (II) or pharmaceutically acceptable salts thereof can be usually administered orally or parenterally as an injection or the like when used for the above purpose. The effective dose and administration schedule vary depending on the mode of administration, the patient's age, body weight and symptoms, etc., but it is generally preferred to administer them in a dose of 0.01 to 20 mg/kg per day.

The pharmacological activity of the pharmaceutical composition of the present invention is more specifically described below by referring to test examples. In Test Examples 1 to 5, hydrochloride of Compound 22 was used as the test compound. The compounds used in combination with the Hsp90 family protein inhibitor in the test examples were obtained as commercial products or synthesized by known methods.

Test Example 1: Analysis of Combination Effect Using in vitro Cell Growth Inhibition Assay and the Isobologram Method

Measurement of the cell growth inhibition rate of the test compound and a combined compound on a human multiple myeloma cell line (NCI-H929), a human non-small-cell lung cancer cell line (PC-9), a human breast cancer cell line (BT-474), a human renal cancer cell line (VMRC-RCZ), a human chronic myeloid leukemia cell line (K-562) and a human acute myeloid leukemia cell line (MOLM-13) was carried out in the following manner.

For the culture of NCI-H929, Roswell Park Memorial Institute's Medium (RPMI) 1640 medium (Invitrogen) containing 10% fetal calf serum (FCS, Invitrogen), 10 mmol/L HEPES (Invitrogen), 1 mmol/L sodium pyruvate (Invitrogen), 4.5 g/L glucose (Sigma-Aldrich) and 50 μmol/L 2-mercaptoethanol (Invitrogen) was used. For the culture of PC-9, RPMI1640 medium containing 10% FCS was used. For the culture of BT-474, Dulbecco's Modified Eagle Medium (DMEM) (Invitrogen) containing 10% FCS, 1 mmol/L sodium pyruvate, 1.2 mmol/L oxalacetic acid (Sigma-Aldrich), 0.01 mg/mL insulin (Sigma-Aldrich), 10% NCTC-135 medium (Sigma-Aldrich), 4 mmol/L L-glutamine (Invitrogen), 1.5 g/L sodium hydrogencarbonate (Invitrogen) and 4.5 g/L glucose was used.

For the culture of VMRC-RCZ, Minimum Essential Medium (MEM) (Invitrogen) containing 10% FCS, 0.1 mmol/L Non-Essential Amino Acids (NEAA, Invitrogen), 4 mmol/L HEPES, 2 mmol/L L-glutamine and 1.5 g/L sodium hydrogencarbonate was used. For the culture of K-562, Iscove's Modified Dulbecco's Medium (IMDM) (Invitrogen) containing 10% FCS was used. For the culture of MOLM-13, RPMI1640 medium containing 10% FCS was used.

NCI-H929 cells were suspended to 12.5 x $10^4$ cells/mL with culture medium (PC-9, 0.625 x $10^4$ cells/mL; BT-474, 12.5 x $10^4$ cells/mL; VMRC-RCZ, 2.5 x $10^4$ cells/mL; MOLM-13, 12.5 x $10^4$ cells/mL; K-562, 6.25 x $10^4$ cells/mL), and the cell suspensions were seeded to the wells of 96-well U-bottom plates (Nalge Nunc International) in an amount of 80 μL/well, followed by incubation in a 5% $CO_2$ incubator at 37°C for 5 hours (PC-9, BT-474 and VMRC-RCZ, 24 hours; MOLM-13 and K-562, 4 hours).

The solutions of the test compound and the combined compound diluted with culture medium were added to the plates in an amount of 10 μL/well, respectively, followed by further incubation in a 5% $CO_2$ incubator at 37°C for 72 hours. WST-1 reagent {4-[3-(4-iodophenyl)-2-(4-nitrophenyl)-2H-5-tetrazolio]-1,3-benzene disulfonate sodium salt} (Roche Diagnostics) was added to the wells in an amount of 10 μL/well and the plates were incubated in a $CO_2$ incubator for 2 hours at 37°C. The absorbance at 450 nm (control wavelength: 655 nm) was measured using a microplate spectrophotometer SPECTRA max 340PC (Molecular Devices). Separately, the solvent used for preparation of each compound solution alone was added to the wells, and the absorbance was measured in the same manner as above after 72-hour incubation carried out in the same manner as above and also immediately after the addition of the solvent. The cell growth inhibition rate on the cells in the compound-added groups (groups treated with the test compound alone, the combined compound alone, or both compounds) was calculated according to the following equation.

$$\text{Cell growth inhibition rate (\%)} = 100 - \left( \frac{\text{absorbance at 72 hours after addition of test compound} - \text{absorbance immediately after addition of compound}}{\text{absorbance at 72 hours after addition of solvent} - \text{absorbance immediately after addition of solvent}} \right) \text{X } 100$$

By using the above method, the concentrations showing 5 to 50% cell growth inhibition ($IC_5$ to $IC_{50}$) when the test compound alone or the combined compound alone was added and $IC_{50}$ when both the test compound and the combined compound were added were calculated. The combined effect was analyzed using the isobologram method [International Journal of Radiation Oncology, Biology, Physics, Vol. 5, p. 85 (1979)]. Judgment of the efficacy of combination therapy was carried out according to the method described in International Journal of Radiation Oncology, Biology, Physics, Vol. 5, p. 85 (1979), Ibid., Vol. 5, p. 1145 (1979), etc. A combination of compounds judged to be supra-additive (synergistic effect), envelope of additivity (additive effect) or sub-additive (additive tendency) was judged as having combination effect, and a combination of compounds judged to be protection (antagonistic action) was judged as not having combination effect. The combination effect of the test compound and each combined compound on the cells is shown in Table 3.

The compounds used as the combined compounds are bortezomib, melphalan, dexamethasone, lenalidomide, rapamycin, vorinostat, gefitinib, erlotinib, paclitaxel, docetaxel, trastuzumab, lapatinib, cytarabine, sorafenib, sunitinib, fludarabine and imatinib. All of the combinations of the test compound and the combined compound were judged as having combination effect.

From the foregoing, it was revealed that the combinations of the test compound and the combined compounds shown in Table 3 exhibit enhanced cell growth inhibiting effect compared with either compound alone. This result suggests that the combination use of an Hsp90 family protein inhibitor and an antitumor agent has a higher cell growth inhibiting activity than the single administration of either agent.

Table 3: Combination effect of test compound and each compound on each cell line

| Cell line | Combined compound | Combinztion effect |
|---|---|---|
| NCI-H929 | Bortezomib | Envelope of additivity |
| | Melphalan | Envelope of additivity |
| | Dexamethasone | Envelope of additivity |
| | Lenalidomide | Envelope of additivity |
| | Rapamycin | Envelope of additivity |
| | Vorinostat | Sub-additive |
| PC-9 | Gefitinib | Envelope of additivity |
| | Erlotinib | Sub-additive |
| | Paclitaxel | Envelope of additivity |
| | Docetaxel | Envelope of additivity |
| BT-474 | Trastuzumab | Envelope of additivity |
| | Lapatinib | Envelope of additivity |

(continued)

| Cell line | Combined compound | Combinztion effect |
|---|---|---|
| VMRC-RCZ | Sorafenib | Supra-additive |
| | Sunitinib | Supra-additive |
| MOLM-13 | Cytarabine | Envelope of additivity |
| | Fludarabine | Envelope of additivity |
| K-562 | Imatinib | Sub-additive |

Test Example 2: Antitumor Effect of Combination of an Hsp90 Family Protein Inhibitor and Gefitinib in a Mouse Model Transplanted with Human Lung Cancer NCI-H596 Cells

NCI-H596 cells were cultured in RPMI1640 medium containing 10% fetal calf serum (FCS) in a 5% $CO_2$ incubator at 37˚C and then transplanted into the abdominal subcutaneous tissue of BALB/cAJcl-nu mice (Clea Japan, Inc.) in an amount of 1 x $10^7$ cells/mouse. From the mice in which tumor grew, the tumor was excised and the tumor tissue was cut into ca. 8 $mm^3$ tissue pieces and then transplanted into the abdominal subcutaneous tissue of BALB/cAJcl-nu mice (Clea Japan, Inc.) using a trocar. Seventeen days after the transplantation, the longer diameter and shorter diameter of the subcutaneous tumor were measured with slide calipers, and the tumor volume was calculated according to the following equation.

$$\underset{(mm^3)}{\text{Tumor volume}} = \underset{(mm)}{\text{major axis}} \times \underset{(mm)}{\text{minor axis}} \times \underset{(mm)}{\text{minor axis}} \times \frac{1}{2}$$

At the same time, the body weight of each mouse was measured, and the mice were divided into the following groups of 5 mice each so that the mean tumor volume and the mean body weight become uniform. The day was defined as day 0 of the administration test, and administration was started.

The test compound was dissolved in physiological saline (Otsuka Pharmaceutical Co., Ltd.) at a concentration of 10 mg/mL and intravenously administered from the caudal vein in a dose of 0.01 mL/g body weight of mouse (100 mg/kg) twice a day for consecutive days from day 0 to day 4.

Gefitinib was suspended in physiological saline containing 0.5% Tween 80 at a concentration of 10 mg/mL and orally administered in a dose of 0.01 mL/g body weight of mouse (100 mg/kg) once a day for consecutive days from day 0 to day 4.

A. Negative control group (Control): neither test compound nor gefitinib administered
B. Test compound alone group: 100 mg/kg (twice a day x 5 days)
C. Gefitinib alone group: 100 mg/kg (once a day x 5 days)
D. Test compound + gefitinib: test compound, 100 mg/kg (twice a day x 5 days); gefitinib, 100 mg/kg (once a day x 5 days)

On and after day 0, the tumor volume was measured twice a week. Judgment of antitumor effect was carried out by calculating the mean tumor volume of each group and comparing the change in relative tumor volume (V/V0) based on the tumor volume at day 0 (V0). The V/V0 chronologically measured for each group is shown in Fig. 1.

As shown in Fig. 1, the combined administration of the test compound and gefitinib exhibited a higher growth inhibitory effect than the single administration of either the test compound or gefitinib.

The value obtained by dividing the V/V0 at day 11 of each group by the V/V0 of the negative control group (T/C) is shown in Table 4. In comparison with the theoretical value of T/C obtained by simply adding the drug efficacy of the test compound to that of gefitinib, that is, the value obtained by multiplying the T/C of one of the single administration groups by that of the other, the T/C of the combined administration group actually obtained (D in the table) was lower (0.26) than the theoretical value (0.34) at day 11.

Table 4: T/C of each group

| A. Negative control | B. Test compound | C. Gefitinib | D. Test compound + gefitinib | Theoretical value (B x C) |
|---|---|---|---|---|
| 1 | 0.46 | 0.74 | 0.26 | 0.34 |

From the foregoing, it was revealed that the combined administration of the test compound and gefitinib has a higher antitumor effect than the single administration of either compound and shows synergistic effect. This result suggests that the combined administration of an Hsp90 family protein inhibitor and a molecular target drug has a higher antitumor activity than the single administration of either agent and shows synergistic effect.

Test Example 3: Antitumor Effect of Combination of an Hsp90 Family Protein Inhibitor and Paclitaxel or Trastuzumab in a Mouse Model

Transplanted with Human Breast Cancer KPL-4 Cells

KPL-4 cells were cultured in DMEM containing 10% fetal calf serum (FCS) in a 5% $CO_2$ incubator at 37˚C and then transplanted into the abdominal subcutaneous tissue of BALB/cAJcl-nu mice (Clea Japan, Inc.) in an amount of 1 x $10^7$ cells/mouse. From the mice in which tumor grew, the tumor was excised and the tumor tissue was cut into ca. 8 $mm^3$ tissue pieces and then transplanted into the abdominal subcutaneous tissue of BALB/cAJcl-nu mice (Clea Japan, Inc.) using a trocar. Eighteen days after the transplantation, the longer diameter and shorter diameter of the subcutaneous tumor were measured with slide calipers, and the tumor volume was calculated according to the following equation.

$$\underset{(mm^3)}{\text{Tumor volume}} = \underset{(mm)}{\text{major axis}} \times \underset{(mm)}{\text{minor axis}} \times \underset{(mm)}{\text{minor axis}} \times \frac{1}{2}$$

At the same time, the body weight of each mouse was measured, and the mice were divided into the following groups of 5 mice each so that the mean tumor volume and the mean body weight become uniform. The day was defined as day 0 of the administration test, and administration was started.

The test compound was dissolved in physiological saline (Otsuka Pharmaceutical Co., Ltd.) at a concentration of 10 mg/mL and intravenously administered from the caudal vein in a dose of 0.01 mL/g body weight of mouse (100 mg/kg) twice a day for consecutive days from day 0 to day 4.

Paclitaxel was dissolved in an administration solvent [a solution in which N,N-dimethylacetamide (Wako Pure Chemical Industries, Ltd.), CREMOPHOR EL (Sigma-Aldrich Corp.) and physiological saline (Otsuka Pharmaceutical Co., Ltd.) were mixed at a ratio by volume of 1:1:8] at a concentration of 2.5 mg/mL and intravenously administered from the caudal vein in a dose of 0.01 mL/g body weight of mouse (25 mg/kg) once a day on day 0 and day 3.

Trastuzumab was dissolved in an administration solvent [a solution in which distilled water and physiological saline (Otsuka Pharmaceutical Co., Ltd.) were mixed at a ratio by volume of 1:3.17] at a concentration of 5 mg/mL and intravenously administered from the caudal vein in a dose of 0.01 mL/g body weight of mouse (50 mg/kg) once a day on days 0, 3, 7, 10, 14 and 17.

A. Negative control group (Control): none of the test compound, paclitaxel and trastuzumab administered
B. Test compound alone group: 100 mg/kg (twice a day x 5 days)
C. Paclitaxel alone group: 25 mg/kg (once a day/administered on days 0 and 3)
D. Test compound + paclitaxel: test compound, 100 mg/kg (twice a day x 5 days); paclitaxel, 25 mg/kg (once a day/administered on days 0 and 3)
E. Trastuzumab alone group: 50 mg/kg (once a day/administered on days 0, 3, 7, 10, 14 and 17)
F. Test compound + trastuzumab: test compound, 100 mg/kg (twice a day x 5 days); trastuzumab, 50 mg/kg (once a day/administered on days 0, 3, 7, 10, 14 and 17)

On and after day 0, the tumor volume was measured twice a week. Judgment of antitumor effect was carried out by calculating the mean tumor volume of each group and comparing the change in relative tumor volume (V/V0) based on the tumor volume at day 0 (V0). The V/V0 chronologically measured for each group is shown in Fig. 2 or Fig. 3.

As shown in Fig. 2, the combined administration of the test compound and paclitaxel exhibited a higher growth inhibitory effect than the single administration of either the test compound or paclitaxel.

The value obtained by dividing the V/V0 at day 10 of each group by the V/V0 of the negative control group (T/C) is shown in Table 5. In comparison with the theoretical value of T/C obtained by simply adding the drug efficacy of the test compound to that of paclitaxel, that is, the value obtained by multiplying the T/C of one of the single administration groups by that of the other, the T/C of the combined administration group actually obtained (D in the table) was lower (0.042) than the theoretical value (0.065) at day 10.

Table 5: T/C of each group

| A. Negative control | B. Test compound | C. Paclitaxel | D. Test compound + paclitaxel | Theoretical value (B x C) |
|---|---|---|---|---|
| 1 | 0.69 | 0.094 | 0.042 | 0.065 |

As shown in Fig. 3, the combined administration of the test compound and trastuzumab exhibited a higher growth inhibitory effect than the single administration of either the test compound or trastuzumab.

The value obtained by dividing the V/V0 at day 10 of each group by the V/V0 of the negative control group (T/C) is shown in Table 6. In comparison with the theoretical value of T/C obtained by simply adding the drug efficacy of the test compound to that of trastuzumab, that is, the value obtained by multiplying the T/C of one of the single administration groups by that of the other, the T/C of the combined administration group actually obtained (F in the table) was lower (0.35) than the theoretical value (0.66) at day 10.

Table 6: T/C of each group

| A. Negative control | B. Test compound | E. Trastuzumab | F. Test compound + trastuzumab | Theoretical value (B x E) |
|---|---|---|---|---|
| 1 | 0.69 | 0.96 | 0.35 | 0.66 |

From the foregoing, it was revealed that the combined administration of the test compound and paclitaxel or trastuzumab has a higher antitumor effect than the single administration of either compound and shows synergistic effect. This result suggests that the combined administration of an Hsp90 family protein inhibitor and a tubulin acting agent or an anti-ErbB2 antibody has a higher antitumor activity than the single administration of either agent and shows synergistic effect.

<u>Test Example 4: Antitumor Effect of Combination of an Hsp90 Family Protein Inhibitor and Bortezomib in a Mouse Model Transplanted with Human Multiple Myeloma NCI-H929 Cells</u>

On the day before the transplantation of cancer cells, 0.3 mg/mouse of an anti-asialo GM1 antibody (ca. 10 mg protein/1 mL vial) (Wako Pure Chemical Industries, Ltd.) was intraperitoneally administered to Fox C.B-17/Icr-scidJcl mice (Clea Japan, Inc.). NCI-H929 cells were cultured in RPMI1640 medium containing 10% fetal calf serum (FCS) in a 5% $CO_2$ incubator at 37˚C and then transplanted into the abdominal subcutaneous tissue of the mice in an amount of 1 x $10^7$ cells/mouse. Ten days after the transplantation, the longer diameter and shorter diameter of the tumor subcutaneously grown were measured with slide calipers, and the tumor volume was calculated according to the following equation.

$$\underset{(mm^3)}{\text{Tumor volume}} = \underset{(mm)}{\text{major axis}} \times \underset{(mm)}{\text{minor axis}} \times \underset{(mm)}{\text{minor axis}} \times \frac{1}{2}$$

At the same time, the body weight of each mouse was measured, and the mice were divided into the following groups of 5 mice each so that the mean tumor volume and the mean body weight become uniform. The day was defined as day 0 of the administration test, and administration was started.

The test compound was dissolved in physiological saline (Otsuka Pharmaceutical Co., Ltd.) at a concentration of 5 mg/mL and intravenously administered from the caudal vein in a dose of 0.01 mL/g body weight of mouse (50 mg/kg) once a day on days 0, 3, 7 and 10.

Bortezomib was suspended in physiological saline (Otsuka Pharmaceutical Co., Ltd.) at a concentration of 0.1 mg/mL and intravenously administered from the caudal vein in a dose of 0.01 mL/g body weight of mouse (1 mg/kg)

once a day on days 0, 3, 7 and 10.

A. Negative control group (Control): neither test compound nor bortezomib administered
B. Test compound alone group: 50 mg/kg (once a day/administered on days 0, 3, 7 and 10)
C. Bortezomib alone group: 1 mg/kg (once a day/administered on days 0, 3, 7 and 10)
D. Test compound + bortezomib: test compound, 50 mg/kg; bortezomib, 1 mg/kg (each administered once a day/on days 0, 3, 7 and 10)

On and after day 0, the tumor volume was measured twice a week. Judgment of antitumor effect was carried out by calculating the mean tumor volume of each group and comparing the change in relative tumor volume (V/V0) based on the tumor volume at day 0 (V0). The V/V0 chronologically measured for each group is shown in Fig. 4.
As shown in Fig. 4, the combined administration of the test compound and bortezomib exhibited a higher growth inhibitory effect than the single administration of either the test compound or bortezomib.
The value obtained by dividing the V/V0 at day 14 of each group by the V/V0 of the negative control group (T/C) is shown in Table 7. In comparison with the theoretical value of T/C obtained by simply adding the drug efficacy of the test compound to that of bortezomib, that is, the value obtained by multiplying the T/C of one of the single administration groups by that of the other, the T/C of the combined administration group actually obtained (D in the table) was lower (0.084) than the theoretical value (0.14) at day 14.

Table 7: T/C of each group

| A. Negative control | B. Test compound | C. Bortezomib | D. Test compound + bortezomib | Theoretical value (B x C) |
|---|---|---|---|---|
| 1 | 0.31 | 0.45 | 0.084 | 0.14 |

From the foregoing, it was revealed that the combined administration of the test compound and bortezomib has a higher antitumor effect than the single administration of either compound and shows synergistic effect. This result suggests that the combined administration of an Hsp90 family protein inhibitor and a molecular targeted drug has a higher antitumor activity than the single administration of either agent and shows synergistic effect.

Test Example 5: Antitumor Effect of Combination of an Hsp90 Family Protein Inhibitor and Melphalan in a Mouse Model Transplanted with Human Multiple Myeloma NCI-H929 Cells

On the day before the transplantation of cancer cells, 0.3 mg/mouse of an anti-asialo GM1 antibody (ca. 10 mg protein/1 mL vial) (Wako Pure Chemical Industries, Ltd.) was intraperitoneally administered to Fox C.B-17/Icr-scidJcl mice (Clea Japan, Inc.). NCI-H929 cells were cultured in RPMI1640 medium containing 10% fetal calf serum (FCS) in a 5% $CO_2$ incubator at 37°C and then transplanted into the abdominal subcutaneous tissue of the mice in an amount of 1 x $10^7$ cells/mouse. Ten days after the transplantation, the longer diameter and shorter diameter of the tumor subcutaneously grown were measured with slide calipers, and the tumor volume was calculated according to the following equation.

$$\underset{(mm^3)}{\text{Tumor volume}} = \underset{(mm)}{\text{major axis}} \times \underset{(mm)}{\text{minor axis}} \times \underset{(mm)}{\text{minor axis}} \times \frac{1}{2}$$

At the same time, the body weight of each mouse was measured, and the mice were divided into the following groups of 5 mice each so that the mean tumor volume and the mean body weight become uniform. The day was defined as day 0 of the administration test, and administration was started.
The test compound was dissolved in physiological saline (Otsuka Pharmaceutical Co., Ltd.) at a concentration of 5 mg/mL and intravenously administered from the caudal vein in a dose of 0.01 mL/g body weight of mouse (50 mg/kg) once a day on days 0, 3, 7 and 10.
Melphalan was suspended in physiological saline (Otsuka Pharmaceutical Co., Ltd.) at a concentration of 0.4 mg/mL and intravenously administered from the caudal vein in a dose of 0.01 mL/g body weight of mouse (4 mg/kg) once a day on day 0.

A. Negative control group (Control): neither test compound nor melphalan administered
B. Test compound alone group: 50 mg/kg (once a day/administered on days 0, 3, 7 and 10)
C. Melphalan alone group: 4 mg/kg (once a day x one day)
D. Test compound + melphalan: test compound, 50 mg/kg (once a day/administered on days 0, 3, 7 and 10); melphalan, 4 mg/kg (once a day x one day)

On and after day 0, the tumor volume was measured twice a week. Judgment of antitumor effect was carried out by calculating the mean tumor volume of each group and comparing the change in relative tumor volume (V/V0) based on the tumor volume at day 0 (V0). The V/V0 chronologically measured for each group is shown in Fig. 5.
As shown in Fig. 5, the combined administration of the test compound and melphalan exhibited a higher growth inhibitory effect than the single administration of either the test compound or melphalan.
The value obtained by dividing the V/V0 at day 14 of each group by the V/V0 of the negative control group (T/C) is shown in Table 8. In comparison with the theoretical value of T/C obtained by simply adding the drug efficacy of the test compound to that of melphalan, that is, the value obtained by multiplying the T/C of one of the single administration groups by that of the other, the T/C of the combined administration group actually obtained (D in the table) was lower (0.11) than the theoretical value (0.28) at day 14.

Table 8: T/C of each group

| A. Negative control | B. Test compound | C. Melphalan | D. Test compound + melphalan | Theoretical value (B x C) |
|---|---|---|---|---|
| 1 | 0.45 | 0.63 | 0.11 | 0.28 |

From the foregoing, it was revealed that the combined administration of the test compound and melphalan has a higher antitumor effect than the single administration of either compound and shows synergistic effect. This result suggests that the combined administration of an Hsp90 family protein inhibitor and a DNA acting agent has a higher antitumor activity than the single administration of either agent and shows synergistic effect.

Example 1

Preparation Example 1 (Tablet)

Tablet having the following formulation is prepared in a conventional manner.

| | |
|---|---|
| Compound 1 | 5 mg |
| Lactose | 60 mg |
| Potato starch | 30 mg |
| Poly(vinyl alcohol) | 2 mg |
| Magnesium stearate | 1 mg |
| Tar pigment | trace amount |

Example 2

Preparation Example 2 (Tablet)

Tablet having the following formulation is prepared in a conventional manner.

| | |
|---|---|
| Compound 11 | 5 mg |
| Gefitinib | 10 mg |
| Lactose | 60 mg |
| Potato starch | 30 mg |
| Poly(vinyl alcohol) | 2 mg |
| Magnesium stearate | 1 mg |
| Tar pigment | trace amount |

Example 3

Preparation Example 3 (Injection)

Injection having the following formulation is prepared in a conventional manner.

| | |
|---|---|
| Compound 17 | 2 mg |
| D-mannitol | 10 mg |

Aqueous hydrochloric acid solution proper quantity Aqueous sodium hydroxide solution proper quantity Distilled water for injection proper quantity

Example 4

Preparation Example 4 (Injection)

Injection having the following formulation is prepared in a conventional manner.

| | |
|---|---|
| Trastuzumab | 2 mg |
| D-mannitol | 10 mg |
| Aqueous hydrochloric acid solution | proper quantity |
| Aqueous sodium hydroxide solution | proper quantity |
| Distilled water for injection | proper quantity |

Industrial Applicability

The present invention provides a pharmaceutical composition comprising a combination of an Hsp90 family protein inhibitor and at least one compound, and the like.

## Claims

1. A pharmaceutical composition comprising a combination of a Heat shock protein 90 (Hsp90) family protein inhibitor and at least one compound.

2. A pharmaceutical composition for administering a combination of an Hsp90 family protein inhibitor and at least one compound.

3. A pharmaceutical composition for administering an Hsp90 family protein inhibitor and at least one compound simultaneously or successively.

4. The pharmaceutical composition according to any of claims 1 to 3, wherein the Hsp90 family protein inhibitor is a benzoyl compound represented by formula (I):

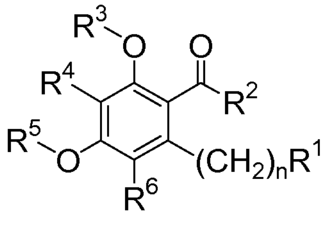

(I)

[wherein n represents an integer of 1 to 5;

$R^1$ represents substituted or unsubstituted lower alkyl, substituted or unsubstituted lower alkoxy, substituted or unsubstituted cycloalkyl, substituted or unsubstituted lower alkoxycarbonyl, substituted or unsubstituted heterocycle-alkyl, substituted or unsubstituted aryl, $CONR^7R^8$ (wherein $R^7$ and $R^8$, which may be the same or different, each represent a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, or substituted or unsubstituted aroyl, or $R^7$ and $R^8$ form a substituted or unsubstituted heterocyclic group together with the adjacent nitrogen atom thereto) or $NR^9R^{10}$ (wherein $R^9$ and $R^{10}$ have the same meanings as the above $R^7$ and $R^8$, respectively);
$R^2$ represents substituted or unsubstituted aryl or a substituted or unsubstituted aromatic heterocyclic group;
$R^3$ and $R^5$, which may be the same or different, each represent a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted aralkyl, or substituted or unsubstituted aroyl;
$R^4$ represents a hydrogen atom, hydroxy or halogen; and
$R^6$ represents a hydrogen atom, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted lower alkanoyl, substituted or unsubstituted aryloxy, substituted or unsubstituted aryl, a substituted or unsubstituted heterocyclic group, substituted or unsubstituted aralkyl, or substituted or unsubstituted heterocycle-alkyl; provided that:

(i) when $R^3$ and $R^5$ are methyl, and $R^4$ and $R^6$ are hydrogen atoms, and

(a) when $-(CH_2)_nR^1$ is methoxycarbonylmethyl,
$R^2$ is not a group selected from the group consisting of 2,4,6-trimethoxy-5-methoxycarbonyl-3-nitrophenyl, 3-cyano-2,4,6-trimethoxyphenyl, 5-cyano-2-ethoxy-4,6-dimethoxy-3-nitrophenyl, 2,6-dimethoxyphenyl, 2-chloro-6-methoxyphenyl and 2-chloro-4,6-dimethoxy-5-methoxycarbonyl-3-nitrophenyl,
(b) when $-(CH_2)_nR^1$ is ethoxycarbonylmethyl,
$R^2$ is not 2,4,6-trimethoxy-3-methoxycarbonylphenyl,
(c) when $-(CH_2)_nR^1$ is N,N-dimethylaminomethyl,
$R^2$ is not phenyl;

(ii) when $R^3$, $R^4$, $R^5$ and $R^6$ are hydrogen atoms, and

(a) when $-(CH_2)_nR^1$ is 2-(acetoxymethyl)heptyl, 3-oxopentyl or pentyl,
$R^2$ is not 6-hydroxy-4-methoxy-3-methoxycarbonyl-2-pentylphenyl,
(b) when $-(CH_2)_nR^1$ is 3-oxopentyl,
$R^2$ is not a group selected from the group consisting of 3-benzyloxycarbonyl-6-hydroxy-4-methoxy-2-pentylphenyl and 3-carboxy-6-hydroxy-4-methoxy-2-pentylphenyl,
(c) when $-(CH_2)_nR^1$ is n-propyl,
$R^2$ is not 2,4-dihydroxy-6-[(4-hydroxy-2-oxopyran-6-yl)methyl]phenyl;

(iii) when $R^3$ and $R^4$ are hydrogen atoms, $R^5$ is methyl, $R^6$ is methoxycarbonyl, and $-(CH_2)_nR^1$ is pentyl,
$R^2$ is not a group selected from the group consisting of 6-[2-(acetoxymethyl)heptyl]-2,4-dihydroxyphenyl, 2,4-dihydroxy-6-pentylphenyl and 2,4-dihydroxy-6-(3-oxopentyl)phenyl;
(iv) when $R^3$ and $R^5$ are benzyl, $R^4$ and $R^6$ are hydrogen atoms, and $-(CH_2)_nR^1$ is 3-oxopentyl,
$R^2$ is not a group selected from the group consisting of 6-benzyloxy-4-methoxy-3-methoxycarbonyl-2-pentylphenyl and 6-benzyloxy-3-benzyloxycarbonyl-4-methoxy-2-pentylphenyl;
(v) when $R^3$ is benzyl, $R^4$ is a hydrogen atom, $R^5$ is methyl, $-(CH_2)_nR^1$ is pentyl, and $R^6$ is methoxycarbonyl or benzyloxycarbonyl,
$R^2$ is not 2,4-bis(benzyloxy)-6-(3-oxopentyl)phenyl;
(vi) when $R^3$ and $R^4$ are hydrogen atoms, $R^5$ is methyl, $-(CH_2)_nR^1$ is pentyl, and $R^6$ is carboxy or benzyloxycarbonyl,
$R^2$ is not 2,4-dihydroxy-6-(3-oxopentyl)phenyl; and
(vii) when $R^3$, $R^4$ and $R^6$ are hydrogen atoms, $R^5$ is n-propyl, and $-(CH_2)_nR^1$ is 5-(1,1-dimethylpropyl)-4-(2-hydrobenzotriazol-2-yl)-2-hydroxyphenylmethyl,

$R^2$ is not phenyl],

or a prodrug thereof; or a pharmaceutically acceptable salt thereof.

**5.** The pharmaceutical composition according to claim 4, wherein $R^2$ is a substituted or unsubstituted aromatic heterocyclic group, aryl substituted with 1 to 3 substituents or aryl.

**6.** The pharmaceutical composition according to claim 4, wherein $R^2$ is aryl substituted with 1 to 3 substituents or aryl.

**7.** The pharmaceutical composition according to claim 4, wherein $R^2$ is phenyl substituted with 1 to 3 substituents or phenyl.

**8.** The pharmaceutical composition according to claim 4, wherein $R^2$ is a substituted or unsubstituted aromatic heterocyclic group.

**9.** The pharmaceutical composition according to any of claims 4 to 8, wherein $R^3$ and $R^5$, which may be the same or different, each are a hydrogen atom, substituted or unsubstituted lower alkyl, substituted or unsubstituted lower alkanoyl, substituted or unsubstituted aroyl, or substituted or unsubstituted lower alkenyl.

**10.** The pharmaceutical composition according to any of claims 4 to 8, wherein $R^3$, $R^4$ and $R^5$ are hydrogen atoms.

**11.** The pharmaceutical composition according to any of claims 4 to 10, wherein $R^1$ is $CONR^7R^8$ (wherein $R^7$ and $R^8$ have the same meanings as defined above, respectively).

**12.** The pharmaceutical composition according to any of claims 4 to 10, wherein $R^1$ is $CONR^{7a}R^{8a}$ (wherein $R^{7a}$ and $R^{8a}$, which may be the same or different, each represent a hydrogen atom, substituted or unsubstituted lower alkyl, or substituted or unsubstituted heterocycle-alkyl).

**13.** The pharmaceutical composition according to any of claims 4 to 10, wherein $R^1$ is $CONR^{7b}R^{8b}$ (wherein $R^{7b}$ and $R^{8b}$ form a substituted or unsubstituted heterocyclic group together with the adjacent nitrogen atom thereto).

**14.** The pharmaceutical composition according to any of claims 4 to 10, wherein $R^1$ is substituted or unsubstituted lower alkoxy.

**15.** The pharmaceutical composition according to any of claims 4 to 14, wherein $R^6$ is a hydrogen atom, lower alkyl, halogen or aryl.

**16.** The pharmaceutical composition according to any of claims 4 to 14, wherein $R^6$ is lower alkyl.

**17.** The pharmaceutical composition according to any of claims 4 to 14, wherein $R^6$ is ethyl.

**18.** The pharmaceutical composition according to any of claims 1 to 3, wherein the Hsp90 family protein inhibitor is a benzoyl compound represented by formula (IA):

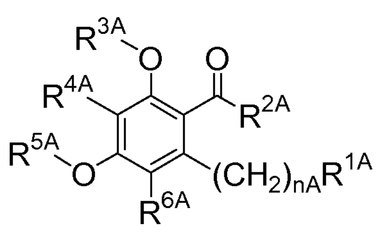
(IA)

[wherein nA represents an integer of 0 to 10;

$R^{1A}$ represents a hydrogen atom, hydroxy, cyano, carboxy, nitro, halogen, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted lower alkanoyloxy, substituted or unsubstituted heterocycle-alkyl, substituted or unsubstituted aryl, substituted or unsubstituted arylsulfonyl, a substituted or unsubstituted heterocyclic group, $CONR^7R^8$ (wherein $R^7$ and $R^8$ have the same meanings as defined above, respectively) or $NR^9R^{10}$ (wherein $R^9$ and $R^{10}$ have the same meanings as defined above, respectively);

$R^{2A}$ represents substituted or unsubstituted lower alkyl, substituted or unsubstituted lower alkenyl, substituted or unsubstituted lower alkynyl, substituted or unsubstituted cycloalkyl, substituted or unsubstituted aryl, or a substituted or unsubstituted heterocyclic group;

$R^{3A}$ and $R^{5A}$, which may be the same or different, each represent a hydrogen atom, substituted or unsubstituted lower alkyl, substituted or unsubstituted lower alkenyl, substituted or unsubstituted lower alkanoyl, substituted or unsubstituted cycloalkyl, substituted or unsubstituted aralkyl, or substituted or unsubstituted aroyl; and

$R^{4A}$ and $R^{6A}$, which may be the same or different, each represent a hydrogen atom, hydroxy, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted lower alkanoyl, substituted or unsubstituted aryloxy, substituted or unsubstituted aryl, a substituted or unsubstituted heterocyclic group, substituted or unsubstituted aralkyl, or substituted or unsubstituted heterocycle-alkyl],

or a prodrug thereof; or a pharmaceutically acceptable salt thereof.

**19.** The pharmaceutical composition according to any of claims 1 to 3, wherein the Hsp90 family protein inhibitor is a benzene derivative represented by formula (II):

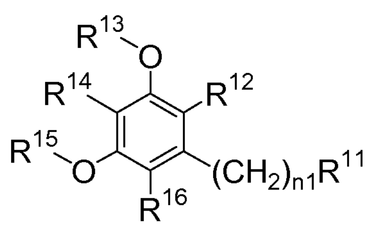

(II)

{wherein n1 represents an integer of 0 to 10;

$R^{11}$ represents a hydrogen atom, hydroxy, cyano, carboxy, nitro, halogen, substituted or unsubstituted lower alkyl, substituted or unsubstituted lower alkenyl, substituted or unsubstituted lower alkynyl, substituted or unsubstituted cycloalkyl, substituted or unsubstituted lower alkoxycarbonyl, substituted or unsubstituted aroyl, substituted or unsubstituted lower alkanoyl, substituted or unsubstituted heterocycle-alkyl, substituted or unsubstituted aryl, substituted or unsubstituted aralkyl, substituted or unsubstituted arylsulfonyl, a substituted or unsubstituted heterocyclic group, $CONR^{17}R^{18}$ (wherein $R^{17}$ and $R^{18}$, which may be the same or different, each represent a hydrogen atom, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, or substituted or unsubstituted aroyl, or $R^{17}$ and $R^{18}$ form a substituted or unsubstituted heterocyclic group together with the adjacent nitrogen atom thereto), $NR^{19}R^{20}$ [wherein $R^{19}$ and $R^{20}$, which may be the same or different, each represent a hydrogen atom, substituted or unsubstituted lower alkylsulfonyl, substituted or unsubstituted lower alkyl, substituted or unsubstituted cycloalkyl, substituted or unsubstituted lower alkanoyl, substituted or unsubstituted aryl, a substituted or unsubstituted heterocyclic group, substituted or unsubstituted aralkyl, substituted or unsubstituted heterocycle-alkyl, substituted or unsubstituted aroyl or $CONR^{21}R^{22}$ (wherein $R^{21}$ and $R^{22}$ have the same meanings as the above $R^{17}$ and $R^{18}$, respectively), or $R^{19}$ and $R^{20}$ form a substituted or unsubstituted heterocyclic group together with the adjacent nitrogen atom thereto], or $OR^{23}$ (wherein $R^{23}$ represents substituted or unsubstituted lower alkyl, substituted or unsubstituted lower alkenyl, substituted or unsubstituted lower alkanoyl, substituted or unsubstituted aryl, a substituted or unsubstituted heterocyclic group, substituted or unsubstituted aralkyl, or substituted or unsubstituted heterocycle-alkyl);

$R^{12}$ represents substituted or unsubstituted lower alkyl, substituted or unsubstituted lower alkenyl, substituted or unsubstituted lower alkynyl, substituted or unsubstituted aryl, or a substituted or unsubstituted heterocyclic group (excluding substituted or unsubstituted pyrazolyl);

$R^{13}$ and $R^{15}$, which may be the same or different, each represent a hydrogen atom, substituted or unsubstituted

lower alkyl, substituted or unsubstituted lower alkenyl, substituted or unsubstituted lower alkanoyl, substituted or unsubstituted cycloalkyl, substituted or unsubstituted lower alkylsulfonyl, substituted or unsubstituted arylsulfonyl, carbamoyl, sulfamoyl, substituted or unsubstituted lower alkylaminocarbonyl, substituted or unsubstituted di-lower alkylaminocarbonyl, substituted or unsubstituted lower alkoxycarbonyl, substituted or unsubstituted heterocycle-carbonyl, substituted or unsubstituted aralkyl, or substituted or unsubstituted aroyl; and $R^{14}$ and $R^{16}$, which may be the same or different, each represent a hydrogen atom, hydroxy, halogen, cyano, nitro, substituted or unsubstituted lower alkyl, substituted or unsubstituted lower alkenyl, substituted or unsubstituted lower alkynyl, substituted or unsubstituted lower alkoxy, substituted or unsubstituted cycloalkyl, amino, lower alkylamino, di-lower alkylamino, carboxy, substituted or unsubstituted lower alkoxycarbonyl, substituted or unsubstituted aryloxy, substituted or unsubstituted aryl, a substituted or unsubstituted heterocyclic group (excluding substituted or unsubstituted pyrazolyl), substituted or unsubstituted lower alkanoyl, substituted or unsubstituted aralkyl, or substituted or unsubstituted heterocycle-alkyl},
or a prodrug thereof; or a pharmaceutically acceptable salt thereof.

**20.** The pharmaceutical composition according to claim 19, wherein $R^{11}$ is a hydrogen atom, hydroxy, cyano, carboxy, nitro, halogen, substituted or unsubstituted lower alkyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted lower alkanoyloxy, substituted or unsubstituted heterocycle-alkyl, substituted or unsubstituted aryl, substituted or unsubstituted arylsulfonyl, $CONR^{17}R^{18}$ (wherein $R^{17}$ and $R^{18}$ have the same meanings as defined above, respectively), or $NR^{19}R^{20}$ (wherein $R^{19}$ and $R^{20}$ have the same meanings as defined above, respectively).

**21.** The pharmaceutical composition according to claim 19, wherein $R^{11}$ is substituted or unsubstituted lower alkyl, substituted or unsubstituted lower alkynyl, substituted or unsubstituted lower alkoxy, substituted or unsubstituted cycloalkyl, substituted or unsubstituted lower alkoxycarbonyl, substituted or unsubstituted heterocycle-alkyl, substituted or unsubstituted aryl, $CONR^{17}R^{18}$ (wherein $R^{17}$ and $R^{18}$ have the same meanings as defined above, respectively), or $NR^{19}R^{20}$ (wherein $R^{19}$ and $R^{20}$ have the same meanings as defined above, respectively).

**22.** The pharmaceutical composition according to any of claims 19 to 21, wherein $R^{12}$ is substituted or unsubstituted aryl or a substituted or unsubstituted aromatic heterocyclic group.

**23.** The pharmaceutical composition according to any of claims 19 to 21, wherein $R^{12}$ is substituted or unsubstituted aryl.

**24.** The pharmaceutical composition according to any of claims 19 to 21, wherein $R^{12}$ is substituted or unsubstituted phenyl.

**25.** The pharmaceutical composition according to any of claims 19 to 21, wherein $R^{12}$ is substituted or unsubstituted furyl.

**26.** The pharmaceutical composition according to any of claims 19 to 25, wherein $R^{14}$ is a hydrogen atom, hydroxy or halogen.

**27.** The pharmaceutical composition according to any of claims 19 to 26, wherein $R^{13}$ and $R^{15}$, which may be the same or different, each are a hydrogen atom, substituted or unsubstituted lower alkyl, substituted or unsubstituted lower alkenyl, substituted or unsubstituted lower alkanoyl, substituted or unsubstituted aroyl, substituted or unsubstituted lower alkylaminocarbonyl, substituted or unsubstituted di-lower alkylaminocarbonyl, substituted or unsubstituted lower alkoxycarbonyl, or substituted or unsubstituted heterocycle-carbonyl.

**28.** The pharmaceutical composition according to any of claims 19 to 25, wherein $R^{13}$, $R^{14}$ and $R^{15}$ are hydrogen atoms.

**29.** The pharmaceutical composition according to any of claims 1 to 28, wherein the target disease is cancer.

**30.** The pharmaceutical composition according to claim 29, wherein the cancer is cancer derived from hematopoietic tumor, breast cancer, uterine body cancer, uterine cervix cancer, prostatic cancer, bladder cancer, renal cancer, gastric cancer, esophageal cancer, hepatic cancer, biliary tract cancer, colon cancer, rectal cancer, pancreatic cancer, lung cancer, oral cavity and pharynx cancer, osteosarcoma, melanoma, or cancer derived from brain tumor.

**31.** The pharmaceutical composition according to claim 29, wherein the cancer is leukemia, myeloma or lymphoma.

**32.** The pharmaceutical composition according to claim 29, wherein the cancer is acute myeloid leukemia.

**33.** The pharmaceutical composition according to claim 29, wherein the cancer is multiple myeloma.

**34.** The pharmaceutical composition according to claim 29, wherein the cancer is solid cancer.

**35.** The pharmaceutical composition according to claim 34, wherein the solid cancer is breast cancer.

**36.** The pharmaceutical composition according to claim 34, wherein the solid cancer is lung cancer.

**37.** The pharmaceutical composition according to any of claims 3 to 36, wherein the compound to be administered in combination, simultaneously or successively, with the Hsp90 family protein inhibitor is a protein or a low-molecular compound.

**38.** The pharmaceutical composition according to claim 37, wherein the compound to be combined with the Hsp90 family protein inhibitor is a protein and the protein is an antibody.

**39.** The pharmaceutical composition according to claim 38, wherein the antibody is an anti-ErbB2 antibody.

**40.** The pharmaceutical composition according to claim 38, wherein the antibody is trastuzumab.

**41.** The pharmaceutical composition according to claim 37, wherein the compound to be combined with the Hsp90 family protein inhibitor is a low-molecular compound and the low-molecular compound is a chemotherapeutic agent or a molecular targeted drug.

**42.** The pharmaceutical composition according to claim 41, wherein the low-molecular compound is a chemotherapeutic agent and the chemotherapeutic agent is melphalan or paclitaxel.

**43.** The pharmaceutical composition according to claim 41, wherein the low-molecular compound is a molecular targeted drug and the molecular targeted drug is a kinase inhibitor.

**44.** The pharmaceutical composition according to claim 43, wherein the kinase inhibitor is gefitinib.

**45.** The pharmaceutical composition according to claim 43, wherein the kinase inhibitor is a fms-like tyrosine kinase 3 (Flt-3) inhibitor.

**46.** The pharmaceutical composition according to claim 43, wherein the kinase inhibitor is an Aurora inhibitor, an Abelson kinase (Abl kinase) inhibitor, a vascular endothelial growth factor receptor (VEGFR) inhibitor, a fibroblast growth factor receptor (FGFR) inhibitor, a platelet derived growth factor receptor (PDGFR) inhibitor or an ephrin inhibitor.

**47.** The pharmaceutical composition according to claim 41, wherein the low-molecular compound is a molecular targeted drug and the molecular targeted drug is a proteasome inhibitor.

**48.** The pharmaceutical composition according to claim 47, wherein the proteasome inhibitor is bortezomib.

**49.** A method of treating cancer, which comprises the step of administering an Hsp90 family protein inhibitor and at least one compound simultaneously or separately with an interval.

**50.** The method of treating cancer according to claim 49, wherein the Hsp90 family protein inhibitor is a benzoyl compound represented by formula (I):

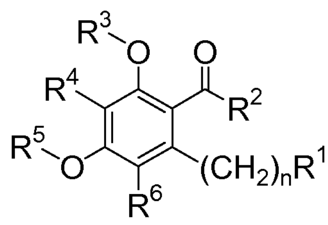

(I)

(wherein n, $R^1$, $R^2$, $R^3$, $R^4$, $R^5$ and $R^6$ have the same meanings as defined above, respectively), or a prodrug thereof; or a pharmaceutically acceptable salt thereof.

**51.** The method of treating cancer according to claim 49, wherein the Hsp90 family protein inhibitor is a benzoyl compound represented by formula (IA):

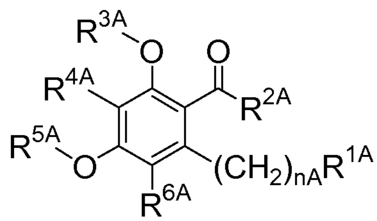

(IA)

(wherein nA, $R^{1A}$, $R^{2A}$, $R^{3A}$, $R^{4A}$, $R^{5A}$ and $R^{6A}$ have the same meanings as defined above, respectively), or a prodrug thereof; or a pharmaceutically acceptable salt thereof.

**52.** The method of treating cancer according to claim 49, wherein the Hsp90 family protein inhibitor is a benzene derivative represented by formula (II):

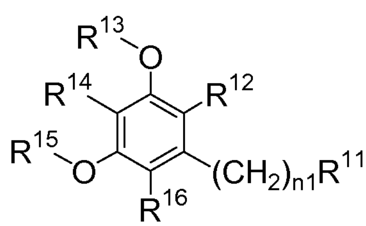

(II)

(wherein n1, $R^{11}$, $R^{12}$, $R^{13}$, $R^{14}$, $R^{15}$ and $R^{16}$ have the same meanings as defined above, respectively), or a prodrug thereof; or a pharmaceutically acceptable salt thereof.

**53.** Use of an Hsp90 family protein inhibitor and at least one compound for the manufacture of an anticancer agent.

**54.** The use according to claim 53, wherein the Hsp90 family protein inhibitor is a benzoyl compound represented by formula (I):

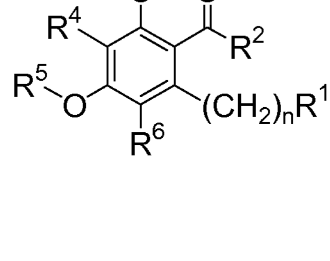

(I)

(wherein n, $R^1$, $R^2$, $R^3$, $R^4$, $R^5$ and $R^6$ have the same meanings as defined above, respectively), or a prodrug thereof; or a pharmaceutically acceptable salt thereof.

**55.** The use according to claim 53, wherein the Hsp90 family protein inhibitor is a benzoyl compound represented by formula (IA) :

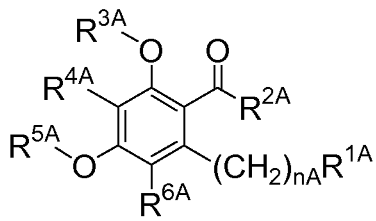

(IA)

(wherein nA, $R^{1A}$, $R^{2A}$, $R^{3A}$, $R^{4A}$, $R^{5A}$ and $R^{6A}$ have the same meanings as defined above, respectively), or a prodrug thereof; or a pharmaceutically acceptable salt thereof.

**56.** The use according to claim 53, wherein the Hsp90 family protein inhibitor is a benzene derivative represented by formula (II) :

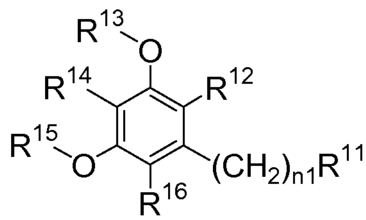

(II)

(wherein n1, $R^{11}$, $R^{12}$, $R^{13}$, $R^{14}$, $R^{15}$ and $R^{16}$ have the same meanings as defined above, respectively), or a prodrug thereof; or a pharmaceutically acceptable salt thereof.

**57.** A kit which comprises a first component comprising an Hsp90 family protein inhibitor and a second component comprising an antitumor agent.

**58.** The kit according to claim 57, wherein the Hsp90 family protein inhibitor is the benzoyl compound described in any of claims 4 to 18, or a prodrug thereof; or a pharmaceutically acceptable salt thereof.

**59.** The kit according to claim 57, wherein the Hsp90 family protein inhibitor is the benzene derivative described in any of claims 19 to 28, or a prodrug thereof; or a pharmaceutically acceptable salt thereof.

**60.** An antitumor agent for administering an Hsp90 family protein inhibitor and at least one compound as active ingredients simultaneously or successively.

**61.** The antitumor agent according to claim 60, wherein the Hsp90 family protein inhibitor is the benzoyl compound described in any of claims 4 to 18, or a prodrug thereof; or a pharmaceutically acceptable salt thereof.

**62.** The antitumor agent according to claim 60, wherein the Hsp90 family protein inhibitor is the benzene derivative described in any of claims 19 to 28, or a prodrug thereof; or a pharmaceutically acceptable salt thereof.

**63.** A method of treating cancer, which comprises the step of applying radiation before or after administering an Hsp90 family protein inhibitor.

Figure 1

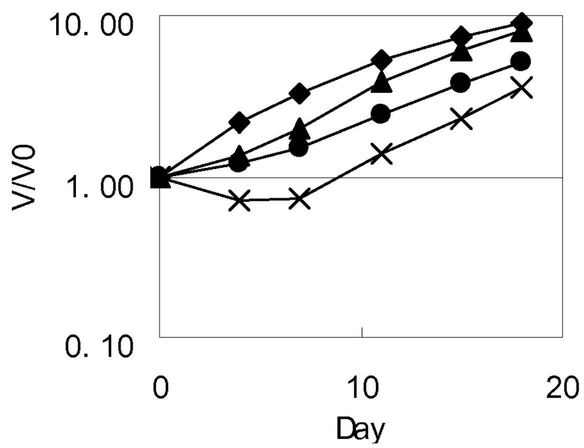


Figure 2

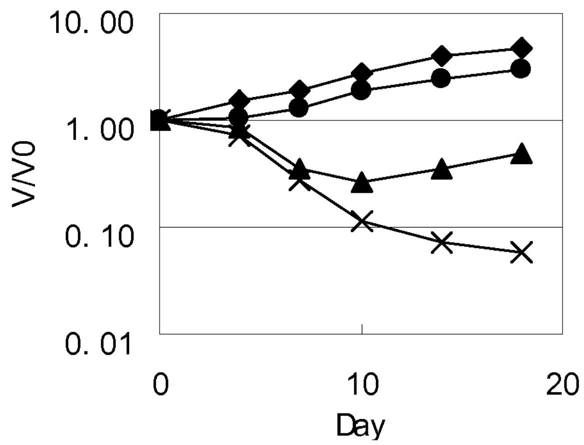

Figure 3

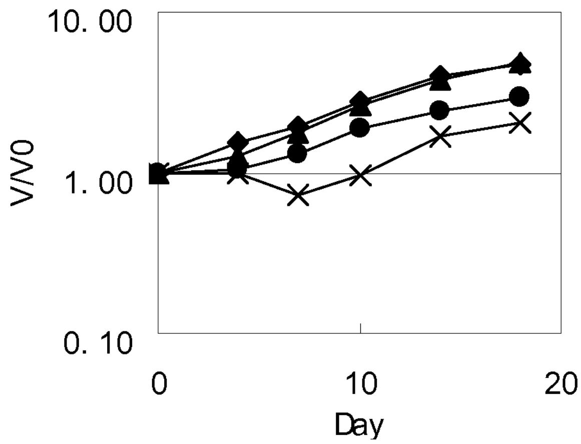


Figure 4

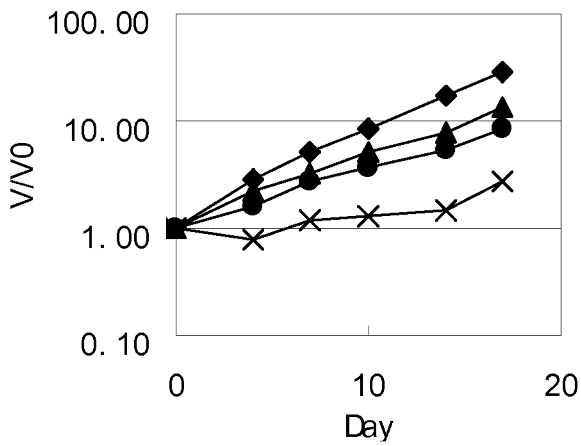

Figure 5

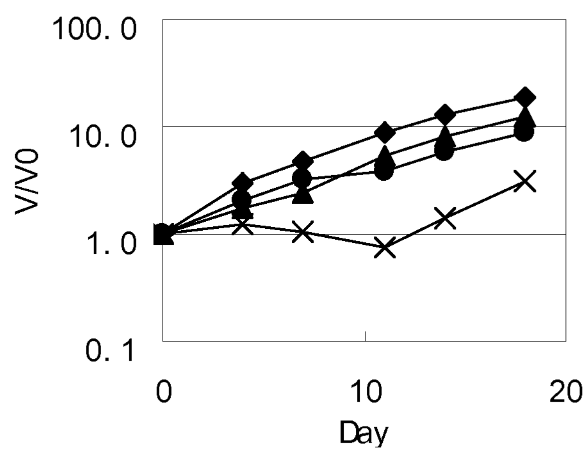

**INTERNATIONAL SEARCH REPORT**

International application No.
PCT/JP2008/053908

A. CLASSIFICATION OF SUBJECT MATTER
*A61K45/06*(2006.01)i, *A61K31/198*(2006.01)i, *A61K31/337*(2006.01)i, *A61K31/5375*(2006.01)i, *A61K31/5377*(2006.01)i, *A61K38/00*(2006.01)i, *A61K39/395*(2006.01)i, *A61P35/00*(2006.01)i, *A61P35/02*(2006.01)i,
According to International Patent Classification (IPC) or to both national classification and IPC

B. FIELDS SEARCHED
Minimum documentation searched (classification system followed by classification symbols)
A61K45/06, A61K31/198, A61K31/337, A61K31/5375, A61K31/5377, A61K38/00, A61K39/395, A61P35/00, A61P35/02, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | WO 2005/000778 A1 (Kyowa Hakko Kogyo Co., Ltd.),<br>06 January, 2005 (06.01.05),<br>Full text; particularly, Claims; examples; test example 1; page 39, 5th line from the bottom to page 40, line 5<br>& US 2007/0032532 A1 & EP 1642880 A1<br>& CA 2530374 A & KR 10-2006-0023576 A<br>& CN 1791568 A | 1-18,29-37,<br>53-55,60,61<br>38-48,57,58 |
| X<br><br>Y | WO 2005/063222 A1 (Kyowa Hakko Kogyo Co., Ltd.),<br>14 July, 2005 (14.07.05),<br>Full text; particularly, Claims; examples; test example 1; page 53, lines 24 to 31<br>& US 2007/0155813 A1 & EP 1704856 A1 | 1-3,19-37,<br>53,56,60,62<br>38-48,57,59 |

[X] Further documents are listed in the continuation of Box C. [ ] See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

Date of the actual completion of the international search
08 May, 2008 (08.05.08)

Date of mailing of the international search report
20 May, 2008 (20.05.08)

Name and mailing address of the ISA/
Japanese Patent Office

Facsimile No.

Authorized officer

Telephone No.

Form PCT/ISA/210 (second sheet) (April 2007)

**INTERNATIONAL SEARCH REPORT**

International application No.
PCT/JP2008/053908

C (Continuation). DOCUMENTS CONSIDERED TO BE RELEVANT

| Category* | Citation of document, with indication, where appropriate, of the relevant passages | Relevant to claim No. |
|---|---|---|
| X<br><br>Y | WO 2006/088193 A1 (Kyowa Hakko Kogyo Co., Ltd.),<br>24 August, 2006 (24.08.06),<br>Full text; particularly, Claims; examples; test examples; Par. Nos. [0046] to [0047]<br>(Family: none) | 1-18,29-37,<br>53-55,60,61<br>38-48,57,58 |
| X<br>Y | WO 2006/010595 A1 (Novartis AG.),<br>02 February, 2006 (02.02.06),<br>Full text; particularly, Claims; examples; page 16, line 17 to page 17, line 17<br>& JP 2008-508218 A & EP 1773327 A1<br>& CA 2574313 A & KR 10-2007-0038565 A | 1-3,53,60<br>38-48,57-59 |
| Y | WO 2005/112952 A2 (KOSAN BIOSCIENCES INC.),<br>01 December, 2005 (01.12.05),<br>Full text; particularly, Claims; page 18, line 17 to page 19, line 2<br>& JP 2008-505984 A & US 2005/0267046 A1<br>& EP 1747005 A2 | 38-48,57-59 |
| Y | Merck Manual, 17th edition, Japanese edition, Nikkei Business Publications, Inc., 10 December, 1999 (10.12.99), pages 983 to 992, chapter 144 Gan Chiryo no Gensoku, particularly, Kagaku Ryoho, table 144-2 | 38-48,57-59 |

Form PCT/ISA/210 (continuation of second sheet) (April 2007)

**INTERNATIONAL SEARCH REPORT**

International application No.
PCT/JP2008/053908

Continuation of A. CLASSIFICATION OF SUBJECT MATTER
(International Patent Classification (IPC))

*A61P43/00*(2006.01)i

(According to International Patent Classification (IPC) or to both national classification and IPC)

Form PCT/ISA/210 (extra sheet) (April 2007)

**INTERNATIONAL SEARCH REPORT**

International application No.
PCT/JP2008/053908

**Box No. II Observations where certain claims were found unsearchable (Continuation of item 2 of first sheet)**

This international search report has not been established in respect of certain claims under Article 17(2)(a) for the following reasons:

1. ☒ Claims Nos.: 49-52, 63
because they relate to subject matter not required to be searched by this Authority, namely:
Claims 49 to 52 and 63 pertain to methods for treatment of the human body by therapy.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.
2. ☐ As all searchable claims could be searched without effort justifying additional fees, this Authority did not invite payment of additional fees.
3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** the

☐ The additional search fees were accompanied by the applicant's protest and, where applicable, payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

**INTERNATIONAL SEARCH REPORT**

International application No.
PCT/JP2008/053908

<Subject of search>

Claims 1-3, 53, 57 and 60 relate to a pharmaceutical composition or an anti-tumor agent comprising a compound defined by a desired property "a heat shock protein 90 family protein inhibitor" as an active ingredient. However, among the compounds having the desired property, those which are supported by the description in the meaning within PCT Article 6 and disclosed in the meaning within PCT Article 5 are limited to a specific extremely small part of the compounds as claimed.

Even though the common technical knowledge at the time of filing the present application is taken into the consideration, it is impossible to specify the scope of the compound having the desired property "a heat shock protein 90 family protein inhibitor".

Further, with regard to the term "prodrug" recited in claims 4, 18, 19 and 54-56, it is unclear as to what compounds having what types of structures are included within the scope of the term.

Such being the case, the search was made mainly on a pharmaceutical composition comprising, as an active ingredient, a compound represented by the formula (I) recited in claim 4 including a hydrochloride of Compound 22 which is the only one compound used in the test examples or a salt (excluding a prodrug) of the compound.

Form PCT/ISA/210 (extra sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02236075 A
- WO 03055860 A
- WO 2005000778 A
- WO 2005063222 A
- WO 2006088193 A
- WO 2005012257 A
- WO 2005012258 A
- WO 2005095382 A
- WO 2005095341 A


### Non-patent literature cited in the description


- *Pharmacol. Ther.,* 1997, vol. 79, 129-168
- *Biochem. Pharmacol.,* 1998, vol. 56, 675-682
- *Invest. New Drugs,* 1999, vol. 17, 361-373
- *Cell Stress Chaperones,* 1998, vol. 3, 100-108
- *J. Med. Chem.,* 1999, vol. 42, 260-266
- *Cell,* 1997, vol. 89, 239-250
- *J. Natl. Cancer Inst.,* 2000, vol. 92, 242-248
- *Cancer Res.,* 1999, vol. 59, 2931-2938
- *Blood,* 2000, vol. 96, 2284-2291
- *Cancer Chemother. Pharmacol.,* 2001, vol. 48, 435-445
- *Current Medicinal Chemistry,* 2007, vol. 14, 223-232
- *Cancer Res.,* 2006, vol. 66, 1089-1095
- *Clin. Cancer Res.,* 2006, vol. 12, 584-590
- *Clin. Cancer Res.,* 2006, vol. 12, 6547-6556
- *Leukemia,* 2006, vol. 20, 610-619
- *Mol. Cancer Thr.,* 2006, vol. 5, 1197-1208
- *Clin. Cancer Res.,* 2004, vol. 10, 8077-8084
- *Clin. Cancer Res.,* 2003, vol. 9, 3749-3755
- *Cancer Res.,* 2003, vol. 63, 2139-2144
- *EMBO Journal,* 2002, vol. 21, 2407-2417
- *Mol. Cancer Thr.,* 2006, vol. 5, 179-186
- *Blood,* 2006, vol. 107, 1092-1100
- *Mol. Cancer Ther.,* 2004, vol. 3, 551-566
- *J. Med. Chem.,* 1997, vol. 40, 2011-2016
- *Drug Dev. Res.,* 1995, vol. 34, 220-230
- *Advances in Drug Res.,* 1984, vol. 13, 224-331
- **Bundgaard.** Design of Prodrugs. Elsevier Press, 1985
- **T.W. Greene.** Protective Groups in Organic Synthesis. John Wiley & Sons Inc, 1999
- *Expert Opinion on Biological Therapy,* 2002, vol. 2, 3-24
- *International Journal of Radiation Oncology, Biology, Physics,* 1979, vol. 5, 85-91
- *International Journal of Radiation Oncology, Biology, Physics,* 1979, vol. 5, 85
- *INTERNATIONAL JOURNAL OF RADIATION ONCOLOGY, BIOLOGY, PHYSICS,* 1979, vol. 5, 1145